(19) **Europäisches Patentamt / European Patent Office / Office européen des brevets**

(11) **EP 3 011 965 B1**

(12) **EUROPEAN PATENT SPECIFICATION**

(45) Date of publication and mention
of the grant of the patent:
**06.05.2020 Bulletin 2020/19**

(51) Int Cl.:
**A61K 36/537** (2006.01)   **A61K 9/16** (2006.01)
**A61P 9/14** (2006.01)

(21) Application number: **14813421.6**

(86) International application number:
**PCT/CN2014/080077**

(22) Date of filing: **17.06.2014**

(87) International publication number:
**WO 2014/201994 (24.12.2014 Gazette 2014/52)**

(54) **SALVIA MILTIORRHIZA EXTRACT, MICROPELLET FORMULATION THEREOF, METHODS OF PREPARING SAME, AND USES THEREOF**

EXTRAKT AUS SALVIA MILTIORRHIZA, MIKROPELLETFORMULIERUNG DARAUS, VERFAHREN ZUR HERSTELLUNG DAVON UND VERWENDUNGEN DAVON

EXTRAIT DE SALVIA MILTIORRHIZA, FORMULATION DE MICROGRANULÉS FORMÉS À PARTIR DUDIT EXTRAIT, PROCÉDÉS DE PRÉPARATION DUDIT EXTRAIT ET SES UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.06.2013   CN 201310237560
17.06.2013   CN 201310237932**

(43) Date of publication of application:
**27.04.2016   Bulletin 2016/17**

(73) Proprietor: **Tasly Pharmaceutical Group Co., Ltd.
Tianjin 300410 (CN)**

(72) Inventors:
• **YAN, Xijun**
  **Tianjin 300410 (CN)**
• **ZHANG, Shunnan**
  **Tianjin 300410 (CN)**
• **YE, Zhengliang**
  **Tianjin 300410 (CN)**
• **ZHOU, Lihong**
  **Tianjin 300410 (CN)**
• **DONG, Hai'ou**
  **Tianjin 300410 (CN)**
• **ZHANG, Wensheng**
  **Tianjin 300410 (CN)**
• **ZHANG, Hongbo**
  **Tianjin 300410 (CN)**
• **MA, Changyu**
  **Tianjin 300410 (CN)**
• **ZHENG, Yongfeng**
  **Tianjin 300410 (CN)**
• **FAN, Lijun**
  **Tianjin 300410 (CN)**

(74) Representative: **Gulde & Partner
Patent- und Rechtsanwaltskanzlei mbB
Wallstraße 58/59
10179 Berlin (DE)**

(56) References cited:
CN-A- 1 827 130       CN-A- 1 827 130
CN-A- 1 943 618       CN-A- 101 040 907
CN-A- 101 773 546     CN-A- 101 773 546
CN-A- 102 133 194     CN-A- 102 229 627
CN-A- 102 772 487     CN-A- 102 846 705
CN-A- 103 127 220

• **CAI ET AL: "Study on Extraction And Refinement of Salvia Miltiorrhiza", CHINESE ARCHIVES OF TRADITIONAL CHINESE MEDI, CN, vol. 11, no. 30, 30 November 2012 (2012-11-30), pages 2565-2570, XP008182635, ISSN: 1673-7717**
• **CAI, WEIWEI.: 'Study on Extraction And Refinement of Salvia Miltiorrhiza' CHINESE ARCHIVES OF TRADITIONAL CHINESE MEDICINE vol. 11, no. 30, 30 November 2012, pages 2565 - 2570, XP008182635**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 011 965 B1

**Description**

**FIELD OF THE INVENTION**

The present invention relates to the field of medicine, more particular to a new Salvia miltiorrhiza extract and its micropellet formulation as well as a micropellet capsulate formulation prepared therefrom. Also, the present invention relates to a formulation containing aforesaid Salvia miltiorrhiza extract, methods of preparing same and uses thereof.

**BACKGROUD OF THE INVENTION**

*Radix salvia miltiorrhiza* (Chinese name: Danshen) is a dried root, which comes from the root of *Salvia militiorrhiza Bunge.* It has been used for treating coronary heart disease (CHD) angina pectoris with nearly thousands of years of history in China. Danshen was first recorded in Classic of *Shen Nong Materia Medica* of E. Han Dynasty and listed in top grade. It is confirmed to have effects of stopping pain by circulating Qi, tranquilizing mind by calming heart, cooling blood by removing heat and removing stasis by activating blood.

Since last century, with the development of chemical separation methods and rising of modern phytochemistry, the chemical constituents of Danshen have been further studied.

As shown in studies, there are mainly two groups of bio-active constituents in Danshen: one category is fat-soluble bio-active constituent represented by Tanshinone and the other is water-soluble bio-active constituent represented by salvianolic acid. Among the fat-soluble bio-active constituents, Tanshinone was proven to have multiple effects of antisepsis, anti-inflammation, removing stasis by activating blood and promoting wound repair. In recent years, studies suggested that the water-soluble bio-active constituent was believed to be the one playing a role in removing stasis by activating blood. For example, salvianolic acid A had a markedly protective effect on I/R-caused myocardial cell injury, while total salvianolic acids showed stronger effect of anti I/R arrhythmia. Salvianolic acid A, salvianolic acid B and total salvianolic acids were confirmed to have protective effects of I/R-caused brain injury in rats, which might reduce MDA content in brain tissue. Not only did the salvianolic acids have effects of anti-thrombosis, but also the protective effect on liver and kidney. Moreover, they had strong effect of anti-oxidation, which might scavenge superoxide anion and free radicals, inhibit lipid peroxidation etc. (Guanhua DU etc, Basic Medical Science and Clinics, 2000, 20(5): 10-14).

Now, extraction method of Danshen water-soluble fraction was focused on the methods of extraction by water and then separation by resin column or polyamide column. For example, Takashi Tanaka etc reported a extraction method of salvianolate (Chemical Pharmaceutical Bulletin, 1989, 37 (2) , 340-344). Moreover, KojiHase etc (PlantaMedica, 1997, 63, 22-26), Yaming XU etc (Chinese Pat. CN1247855A, published on Mar., 2000), Ping LIU etc (Chinese Pat. CN1270809A, published on Oct., 2000) and Lianniang LI etc (Chinese Pat. 01142288.2, published on Sep., 2001) disclosed similar methods of extracting salvianolic acids from Danshen.

In the prior art, the extraction methods of Danshen were generally divided into the following categories: Danshen was extracted with water to have aqueous extraction liquid, which was followed by concentration and ethanol precipitation to give the supernatant. The obtained supernatant was concentrated to obtain the Danshen crude extract with salvianolic acids as the main constituents.

Danshen was decocted with water to have water extraction liquid, which was followed by concentration and ethanol precipitation to give the supernatant. The obtained supernatant was concentrated to a certain degree and passed through macro-porous resin column or polyamide column. The column was eluted with water and ethanol to give the eluent, which was concentrated and dried to have the extract with salvianolic acids as the main constituents.

Danshen was extracted by $CO_2$ supercritical fluid extraction to give tanshinone extract and the residue was decocted with water and precipitated with ethanol to give the supernatant. Afore-obtained supernatant was passed through resin column, which was eluted with water and ethanol to have the water-soluble extract.

In the aforesaid methods, one part of fractions is usually selected as the bio-active constituents, e.g. salvianolic acids or tanshinones. Utilization of Danshen is relatively low, because the other part of bio-active constituents is wasted and not all constituents of Danshen are extracted. This will result in waste of medicine resource.

Blank micropellet is a common carrier in pharmaceutical preparation, which is used in most of commercially available capsules. Firstly, the blank micropellet is loaded with drug and coated to be prepared into the target micropellet having a property of release. It features good fluidity, easy loading into capsule, small filling quantitative difference and stable release etc. Micropellet is widely used in the field of pharmaceutics. As a carrier, it can be both pressed into tablet and loaded into the capsule. Not only does the micropellet improve stability of medicine, but also effectively adjust release rate of medicine. As a drug delivery system, the micropellet has its own advantages therapeutically, e.g. less intestinal stimulation, reduced burst effect of drug and increased drug safety.

Drug-loading technology with the blank micropellet has been used for many years, but very few traditional Chinese medicine (TCM) products are involved. There are more or less shortcomings for TCM drug-loading technology, e.g. small drug loading quantity, instability and poor appearance etc. The patent literature provides few examples of *Salvia militiorrhiza* extract compositions for use in improving mircrocirculation (CN 1 827 130 A), in treatment of cardiovascular diseases (CN 102 846 705 A) or treatment of ischemic conditions (CN 102 772 487 A). The extraction of *Salvia militiorrhiza* may be realized with ethanol and precipitation with water (CN 102 846 705 A); or through additional preparation of salvianolic acid by means of resins from water (CN 102 772 487 A). Non-patent literature such as Cai et a/. ("Study on Extraction And Refinement of Salvia Miltiorrhiza", Chinese Archives of Traditional Chinese Medicine, vol.11, no.30, 2012, p. 2565-2570) provides a comparison between different extraction schemes. A combination of ethanol and water extracts is however not suggested. The subject matter of CN 103 127 220 A and CN 101 773 546 A disclose a fluid bed coating process for *salvia miltiorrhiza*.

## CONTENT OF THE INVENTION

In order to better utilize crude medicine of Danshen, giving full play of pharmacological effect of Danshen, the objective of present invention is to provide a Danshen extract with stronger pharmacological activity. After technology improvement, an appropriate drug-loading technology has been developed for aforesaid Danshen extract, and further prepared into Danshen micropellet.

In this invention, Danshen extract is defined by any one of following numbered paragraph.

1. A Danshen (*salvia miltiorrhiza*) extract, characterized in that said extract comprises following components by weight part: Danshensu : rosmarinic acid : lithospermic acid : salvianolic acid B : cryptotanshinone : tanshinone IIA : stachyose=(0.5-16) : (0.5-15) : (0.5-15) : (5-140) : (0.5-25) : (1-50) : (150-600).

2. The extract according to 1st paragraph, characterized in that said extract comprises following components by weight part: Danshensu : rosmarinic acid : lithospermic acid : salvianolic acid B : cryptotanshinone : tanshinone IIA : stachyose=(1-8) : (1-8) : (1-8) : (10-70) : (1-10) : (2-20) : (250-500).

3. The extract according to 2nd paragraph, characterized in that said extract comprises following components by weight part: Danshensu : rosmarinic acid : lithospermic acid : salvianolic acid B : cryptotanshinone : tanshinone IIA : stachyose=(2-5) : (2-5) : (2-5) : (25-60) : (2-6) : (4-10) : (300-450).

4. The extract according to 3rd paragraph, characterized in that said extract comprises following components by weight part: Danshensu : rosmarinic acid : lithospermic acid : salvianolic acid B : cryptotanshinone : tanshinone IIA : stachyose=(2-4) : (2-4) : (2-4) : (25-30) : (2-5) : (4-10) : (330-400).

5. The extract according to 4th paragraph, characterized in that said extract comprises following components by weight part: Danshensu : rosmarinic acid : lithospermic acid : salvianolic acid B : cryptotanshinone : tanshinone IIA : stachyose=3 : 3 : 3 : 28 : 4 : 7 : 370.

6. A micropellet formulation comprising any one of aforesaid Danshen extracts of 1st~5th paragraphs as active pharmaceutical ingredient (API).

7. The micropellet according to 6th paragraph, characterized in that the bulk density of said micropellet is 0.6~1.3g/ml, specific surface area 0.005-0.05 m²/g and particle size 0.5-1.8mm.

8. The micropellet according to 6th or 7th paragraph, characterized in that the bulk density of said micropellet is 0.8~1.1g/ml, specific surface area 0.01~0.03 m²/g and particle size 0.7-1.2mm.

9. A method of preparing Danshen extract which comprises steps as follows:

(1) Danshen is extracted with alcohol and filtered to give alcohol extraction liquid and residue A for later use;

(2) The residue A is extracted with water, filtered to give water extraction liquid and residue B to be discarded;

(3) Respectively, aforesaid alcohol and water extraction liquids are cooled and allowed to stand still and both supernatants are taken out to give the alcohol and water supernatant respectively;

(4) Aforesaid water supernatant is concentrated to have water concentrated extraction liquid;

(5) The water concentrated extraction liquid is added with the alcohol supernatant gradually, combined and concentrated to give mixed concentrated solution;

(6) The mixed concentrated solution is added with purified water, well blended to give Danshen extract.

10. The method of 9th paragraph, characterized in that in step (1), the alcohol is ethanol at concentration of 50~100% (v/v) in amount of 2~7 times the weight of crude medicine and extraction time 0.5~4 hours.

11. The method of 9th or 10th paragraph, characterized in that in step (2), the amount of water added is 3~7 times the weight of residue A and extraction time 0.5~4 hours.

12. The methods of any one of 9th~11th paragraphs, characterized in that in step (3), said process of cooling and standing still includes steps as follows: the extraction liquid is stirred for 20~60 min and allowed to stand still for 6~24 hours with the temperature decreased to 15°C or lower, before the supernatant is taken out.

13. The methods of any one of 9th~12th paragraphs, characterized in that in step (4), the water supernatant is concentrated to relative density of 1.10~1.35 to give the water concentrated extraction liquid.

14. The methods of any one of 9th~13th paragraphs, characterized in that in step (6), the mixed concentrated solution is added with 10L~100L purified water in several times, 5L~50L each time, blended and concentrated under vacuum to give the extract with relative density of 1.25~1.35 at 82.5±2.5°C.

15. The methods of any one of 9th~14th paragraphs, characterized in that said method comprises steps as follows: Ground Danshen is taken, added with 400±12L ethanol (90±0.5%), decocted for 90±5min, filtered through 200-mesh sieve, blended and put into separation tank. Resultant residue is added with 500±15L water for 2nd decoction of 60±3min, filtered through 200-mesh sieve and the residue is discarded. The water extraction liquid is blended and put into different tanks. Alcohol mixed extraction liquid and water mixed extraction liquid are placed into different separation tanks, which are passed with frozen water to cool and allowed to stand still. After 30 min of stirring, the extraction liquid is cooled to 15°C or lower within 4 hours and allowed to stand still for 6~24 hours. Water supernatant and alcohol supernatant are transferred to respective tanks. Firstly, the water supernatant is concentrated to water concentrated extraction liquid with relative density of 1.25~1.30 (82.5±2.5°C), into which the alcohol supernatant is added gradually and further blended and concentrated. During process of concentration, density of the concentrated solution is not less than 1.15. When the density of mixed concentrated solution is ≥1.34, 10L purified water is added at twice, 5L each time (45±5°C), blended, concentrated to relative density of 1.33~1.35 (82.5±2.5°C), and filtered immediately through 40-mesh sieve to give the Danshen extract.

16. The methods of any one of 9th~15th paragraphs, characterized in that the Danshen extract prepared by said method comprises following components by weight part:
Danshensu : rosmarinic acid : lithospermic acid : salvianolic acid B : cryptotanshinone : tanshinone IIA : stachyose=(0.5-16) : (0.5-15) : (0.5-15) : (5-140) : (0.5-25) : (1-50) : (150-600).

17. The method of 16th paragraph, characterized in that the prepared Danshen extract comprises following components by weight part:
Danshensu : rosmarinic acid : lithospermic acid : salvianolic acid B : cryptotanshinone : tanshinone IIA : stachyose=(1-8) : (1-8) : (1-8) : (10-70) : (1-10) : (2-20) : (250-500).

18. The method of 17th paragraph, characterized in that the prepared Danshen extract comprises following components by weight part:
Danshensu : rosmarinic acid : lithospermic acid : salvianolic acid B : cryptotanshinone : tanshinone IIA : stachyose=(2-5) : (2-5) : (2-5) : (25-60) : (2-6) : (4-10) : (300-450).

19. The method of 18th paragraph, characterized in that the prepared Danshen extract comprises following components by weight part:
Danshensu : rosmarinic acid : lithospermic acid : salvianolic acid B : cryptotanshinone : tanshinone IIA : stachyose=(2-4) : (2-4) : (2-4) : (25-30) : (2-5) : (4-10) : (330-400).

20. The method of 19th paragraph, characterized in that the prepared Danshen extract comprises following components by weight part:
Danshensu : rosmarinic acid : lithospermic acid : salvianolic acid B : cryptotanshinone : tanshinone IIA :

stachyose=3 : 3 : 3 : 28 : 4 : 7 : 370.

21. A method of preparing micropellet containing Danshen extract which comprises steps as follows:

(1) Danshen extract and blank micropellet in a weight ratio of 1:5~5:1 are taken for later use;

(2) Feeding and fluidizing: the blank micropellet of formula dosage is absorbed into fluidized drying coating machine to have the blank micropellet completely fluidized in fluidized-bed;

(3) Drug-loading in early stage: until the material temperature reaches 40-60°C, a liquid is sprayed and spread on the surface of the micropellet at a rate of 70-120g/min. Said liquid as sprayed is small droplets prepared by atomizing premixed Danshen extract obtained by diluting Danshen extract with water;

(4) Drug-loading stage: when the material is at 40~60°C, with increase of diameter of the micropellet, the spray volume is gradually increased. Diameter of micropellet and spraying rate are increased gradiently to give a granule with a diameter of 0.5~1.8mm.

22. The method of 21st paragraph, characterized in that said method additionally comprises step (5): after spraying, the drug-loading micropellet is coated by spraying coating liquid. Temperature of coating material is 40-60°C, spraying rate 40-300g/min, coating time 1~4 hours and concentration of coating liquid 5~25%.

23. The method of 22nd paragraph, characterized in that said method comprises steps as follows:

(1) Danshen extract and blank micropellet in a weight ratio of 1:3~3:1, preferably 2:1~1:1, are taken for later use;

(2) Feeding and fluidizing: at air volume of 600-1500m$^3$/h, the blank micropellet is absorbed into fluidized drying coating machine to have the blank micropellet completely fluidized in fluidized-bed. However, the fluidization cannot be too severe, so as to prevent the blank micropellet from abrading and dusting. Due to this, the blank micropellet should be covered with the droplets atomized by spray gun;

(3) Drug-loading in early stage: spray gun and spray pressure are adjusted to make liquids atomized into small droplet. The extract is delivered to the spray gun by metering pump. The pressures of upper and lower air-jet are respectively set to 2.0~3.0Bar and 2.5~3.5Bar. Temperature of material is at 50°C. Until temperature reaches 45°C, the feed solution begins to spray at a rate of 120g/min;

(4) Drug-loading stage: when the material is at 45~55°C, with increase of diameter of the micropellet, the spray volume is gradually increased, but the maximum rate not more than 400g/min. Air volume is adjusted in accordance with fluidized state of the micropellet;

(5) Coating: after drug loading, coating liquid is directly sprayed to perform coating. Initially, the material temperature is set to 50°C and spraying rate 80g/min. After 20min of coating, the spraying rate can be adjusted to 80-150g/min, material temperature controlled at 40-55°C. Infusion rate, air volume and material temperature are adjusted in accordance with adhesion of micropellet.

24. The method of 23rd paragraph, characterized in that extract medicine solution is prepared by dilution of Danshen extract with water. The weight ratio of Danshen extract to water is 100 : 60-100, preferably 100 : 70-90, more preferably 100 : 75-85.

25. The methods of any one of 21st~24th paragraph, characterized in that said micropellet is the micropellet of any one of 6th~8th paragraph.

26. The Danshen extracts of any one of 1st~5th paragraph or the micropellet formulation of any one of 6th~8th paragraph for use in improving microcirculation or reducing blood lipid.

In this invention, the Danshen extract comprises both water-soluble and fat-soluble constituents of Danshen. By adjustment of process, the water-soluble and fat-soluble constituents are dispersed uniformly in said Danshen extract. On the other hand, concentration sequence and relative density are set in the present method, which will effectively prevent reduced concentration of tanshinone caused by longtime concentration at high temperature. The fat-soluble constituents of Danshen are kept effectively after extracting Danshen with alcohol, which can improve stability of extract. Addition of water in later stage of concentration to concentrate together may reduce residual organic solvents in the extract.

In this invention, as shown in the result of efficacy test, the Danshen extract displayed its effects of significantly improving angiectasia of capillary of auricle microcirculation in mice, further improving microcirculation. On the other hand, as shown in the result of efficacy test, the Danshen extract has the effect of reducing blood lipid.

In this invention, the micropellet formulation features fewer carriers and smaller single dose, usually 0.1~4g each time. Morphologically, it has regular shape of a spherical or sphere-like particle with smooth surface. Said micropellet formulation has excellent physical properties, e.g. good fluidity, well-distributed particle size, compact texture and resistance to compression, resistance to abrasion, high density (bulk density: 0.6-1.3g/ml), small specific surface area (0.005-0.05m²/g, preferably 0.01-0.03m²/g) and short disintegration limit. These properties may not only improve patients' compliance, but also make it possible to promote actual use of coating technology, therefore solving the problems of easily absorbing moisture and instability. Moreover, besides being used as a normal granule, said micropellet of present invention can be used for an immediate product, formula granules or loaded into capsules. In addition, said micropellet can be prepared into various kinds of coating dosage forms, sustainable or controlled release formulation and specific-site drug delivery formulation etc.

## DESCRIPTION OF DRAWINGS

Fig.1 was the comparison diagram of Danshen extracts prepared by two different concentration ways: (1) flask adhesion after discharging Danshen extract; (2) water-washing flask; (3) 95% alcohol-washing flask. Wherein, (a) showed that the alcohol extraction liquid was firstly concentrated and added with the water extraction liquid; (b) showed that water extraction liquid was firstly concentrated and added with alcohol extraction liquid gradually.

Fig.2 was the diagram of coating by two different concentration ways.

Fig.3 was concentration-mortality curve of zebra fish to Danshen extract.

Fig.4 showed the quantitatively analytical region of blood lipid in zebra fish, in which blood lipid was quantitatively analyzed within solid line region.

Fig.5 showed the vascular fat stain in tail of zebra fish 48 hours after treatment by different concentrations.

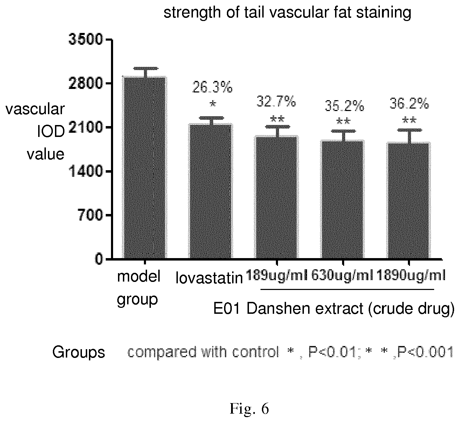

Fig.6 was the bar graph of reduced blood lipid caused by Danshen extract prepared by different concentrations in zebra fish.

## EMBODIMENTS

In an embodiment of this invention, in step (3) of preparative method of Danshen extract, cooling and standing still means that the extract is stirred for 20~60min with its temperature deceased to 15°C or lower, the extract is allowed to stand still for 6~24 hours and supernatant is taken out. By cooling to room temperature or lower, impurities may be removed quickly, e.g. soil particles. Low-temperature can shorten standing time, which is applicable to industrialization, ensuring stability of tanshinone and salvianolic acids.

In an embodiment of this invention, in step (5) of preparative method of Danshen extract, the water concentrated extraction liquid is added with alcohol supernatant of step (3) gradually, combined and concentrated under reduced pressure to give the mixed concentrated liquid with relative density ≥1.20. If alcohol supernatant is firstly concentrated alone, tanshinones will be prone to precipitation and agglomeration as alcohol concentration of liquid decreased, finally adhering to the wall of device. In present method, water concentrated extraction liquid is gradually added with the alcohol supernatant, combined and concentrated, which will make the tanshinones of alcohol supernatant well disperse into water concentrated extraction liquid. Due to this, the extract prepared by the concentration way of present invention is shown to have uniform particle size, which effectively prevent decreased concentration of tanshinones caused by longtime high-temperature concentration and keep a relatively high recovery of fat-soluble constituents prepared by alcohol extraction. In actual production, the extract has simple requirement for cleaning equipment, easy to operate and feasible to be industrialized. More importantly, it will be beneficial to subsequent formulation. Next, the experiments are provided to elaborate the effects of different concentration ways on the properties of Danshen extracts.

In an embodiment of this invention, in step (6) of preparative method of Danshen extract, 10L purified water is added to the mixed concentrated solution at twice, 5L each time, combined, concentrated under reduced pressure to relative density of 1.25-1.35 at 82.5±2.5°C and filtered immediately to give Danshen extract. Addition of water is aimed to evaporate alcohol, control residue alcohol and improve quality of Danshen extract. The product by method of present invention can meet EU requirement for residue alcohol of extract (≤0.5%).

Danshen extract of present invention was assayed by the method disclosed in the prior art.

(1) Determination of tanshinone IIA and cryptotanshinone (referring to items under *Radix salvia miltiorrhiza* in *Chinese Pharmacopoeia* (2010))

Chromatographic conditions and system suitability were present as follows: $C_{18}$ column was used as filling materials; methanol-water (75:25) as mobile phase; detection wavelength at 270nm. The theoretical plate number calculated for tanshinone IIA peak was not lower than 2000.

Preparation of standard solution: appropriate amount of cryptotanshinone and tanshinone IIA were weighed accurately and placed into brown volumetric flask and added with methanol to give the standard solution containing 16μg of cryptotanshinone and tanshinone IIA per ml.

Preparation of sample solution: about 0.2g of Danshen extract was taken, weighed accurately and placed into 10ml volumetric flask. Methanol was added to dissolve by sonication for 30min, cooled to room temperature, continued by addition of methanol to volume, shaken up and passed through 0.22μm organic membrane to give the sample solution.

Assay method: 5μl standard solution and sample solution were respectively drawn with precision, injected into HPLC and measured to give the result.

(2) Determination of salvianolic acids

Chromatographic conditions and system suitability were present as follows: $C_{18}$ column (2.1×100mm, 1.8μm) was used as filling materials; 0.05%(ml/ml) sulfuric acid aqueous solution as mobile phase A, acetonitrile solution as mobile phase B by gradient elution in accordance with Table 1; flow rate at 0.4ml/min; detection wavelength at 280nm, column temperature at 40°C and recording time for 12min. The theoretical plate number calculated for Danshensu peak was not lower than 8000.

Table 1: gradient elution for determination of salvianolic acids

| Time | A (0.05% phosphoric acid solution) (%) | B (acetonitrile) (%) |
|------|------|------|
| 0-1.6 | 93→82 | 7→18 |
| 1.6-1.8 | 82→79.2 | 18→20.8 |
| 1.8-6.1 | 79.2→75 | 20.8→25 |
| 6.1-8.0 | 75→65 | 25→35 |
| 8.0-8.5 | 65→10 | 35→10 |
| 8.5-10.5 | 10 | 90 |
| 10.5-11.0 | 10→93 | 90→7 |
| 11.0-12.0 | 93 | 7 |

Preparation of standard solution: appropriate amount of Danshensu, rosmarinic acid, lithospermic acid and salvianolic acid B were weighed accurately and placed into 100ml volumetric flask. 75% methanol was added to dissolve to the volume and shaken up to give the standard solution at concentration of 0.03, 0.04, 0.04, 0.5mg/ml.

Preparation of sample solution: about 0.1g of sample substance was weighed accurately, placed in 10ml volumetric flask and added with purified water to dissolve by sonication for 15min. Water was added to volume and passed through 0.22μm water membrane to give the sample solution.

Assay method: 2μl standard solution and sample solution were respectively drawn with precision and injected into HPLC to determine by external standard method.

(3) Determination of stachyose

Chromatographic conditions and system suitability
Chromatographic conditions of amino column:
Hypersil-NH2 amino column (4.6 mmx250 mm, 5μm), Dalian Elite, column temperature at 40°C; acetonitrile-water (v:v=70:30) was used as mobile phase; flow rate at 1.0 mL/min.

Preparation of standard solution: appropriate amount of stachyose was weighed accurately and placed in 10ml volumetric flask. Water was added to dissolve to the volume to give the stock solution of stachyose at concentration of 5mg/ml.

Preparation of sample solution: 0.1g of sample substance was weighed accurately, placed in 10ml volumetric flask and added with purified water to dissolve by sonication for 15min. Water was added to volume and passed through 0.22$\mu$m water membrane to give the sample solution.

Assay method: 2$\mu$l standard solution and sample solution were respectively drawn with precision and injected into HPLC to determine by external standard method.

In an embodiment of this invention, the blank micropellet, also known as blank pellet or medicinal pellet, is known in the prior art, which is commercially available. Medicinal pellet cores mainly include medicinal pellet (sucrose type), microcrystalline cellulose pellet and starch pellet etc. Said blank micropellet of the present invention is one selected from starch pellet, PEG-6000 pellet, sucrose pellet or microcrystalline cellulose pellet, preferably starch pellet or PEG-6000 pellet. This pellet shows a series of advantages of high drug-loading, easily coating, easily controlling and feasibility to be industrialized.

In an embodiment of this invention, the drug-loading micropellet can be prepared into pellet capsules after further processing. Said micropellet is coated, sifted to give the coated micropellets, which is loaded into empty capsules to obtain the TCM pellet capsule.

EXAMPLES

The following experimental examples are offered only for the purpose of further illustrating the present invention.

Unless indicated otherwise, % and ‰ in present invention refers to weight ratio.

Experiment 1: effect of different concentration ways on Danshen extract

Following experiments were carried out to investigate effect of different concentration ways on series of properties of trait, particle distribution and salvianolic acid constitution.

450g of Danshen was weighed accurately, extracted with 4 times of 90% ethanol for 1.5 hours and filtered to give the extract solution; resultant residues were refluxed with 5 times of water for 1 hour, filtered, so as to have alcohol extraction liquid and water extraction liquid. Parallel extraction was repeated twice for each experiment.

1st scheme: the alcohol extraction liquid was firstly concentrated, followed by adding with water concentrated extraction liquid gradually, combined and concentrated to give the extract with sugar degree of 86±2%.

2nd scheme: the water extraction liquid was firstly concentrated to sugar degree of 84±2%, followed by adding the alcohol extraction liquid, combined and concentrated to give the extract with sugar degree of 86±2%.

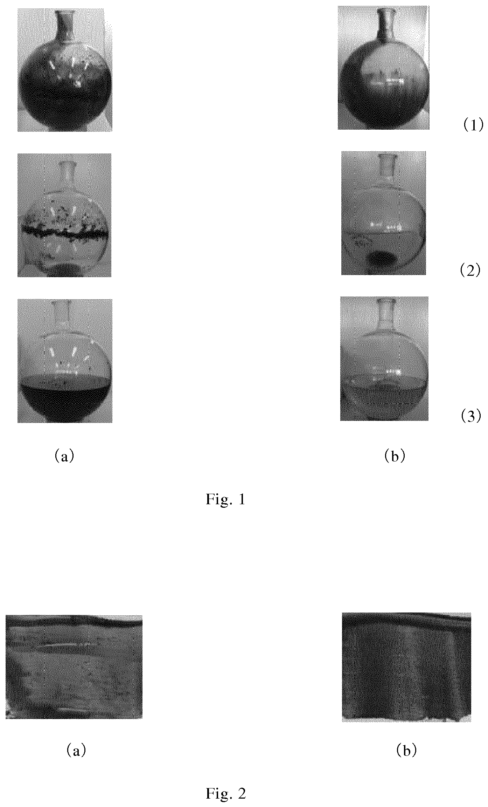

At first, flask adhesion after discharging Danshen extract (Fig. 1 (1)), water-washing flask (Fig. 1 (2)) and 95% alcohol-washing flask (Fig.1 (3)) were investigated to compare advantages and disadvantages between two schemes. Results were obtained, as follows:

As shown in Fig.1 (1), large black slag and flask adhesion were serious in 1st scheme, and the slag was small and uniform in 2nd scheme. The extract by concentration way in 2nd scheme had better character.

As shown in Fig.1 (2), large black slag and flask adhesion were serious in 1st scheme, and adhesion on the wall was less in 2nd scheme. The concentration way in 2nd scheme had a better water-washing effect on the equipment.

As shown in Fig.1 (3), the 95% ethanol-washing liquid was highly colored in 1st scheme, and less colored in 2nd scheme.

To sum up, in terms of appearance character of Danshen extract and subsequent mechanical equipment washing, the concentration way in 2nd scheme is a better option, namely the water extraction liquid was firstly concentrated, followed by adding with alcohol extraction liquid gradually, combined and concentrated.

As shown in Fig.2, seen from coating image of Danshen extract, large and black slag was found to precipitate in Danshen extract by 1st scheme, and small slag was uniformly-distributed in Danshen extract by 2nd scheme, which had less effect on subsequent formulation.

Moreover, particle size distribution on Danshen extracts obtained by two schemes was measured with pharmacopoeia sieve.

Respectively, 50g of Danshen extracts of each batch were taken, into which 5 times water was added to dissolve to give extract liquids. Resultant liquids were passed through #1-8 pharmacopoeia sieves (sieves stacked in sequence), washed with about 500ml distilled water, and particle size distribution on each sieve was observed. Afterwards, the particles on each were washed off, collected, filtered by air pump and drying weighed together with the filter paper. The results were seen in Table 2.

Table 2: analysis of particle size on Danshen extracts by two concentration ways

| Pharmacopoeia sieve | 1st scheme (‰) | 2nd scheme (‰) |
|---|---|---|
| #1 (10 mesh) | 32.98 | - |
| #2 (24 mesh) | 7.58 | - |
| #3 (50 mesh) | 5.57 | - |
| #4 (65 mesh) | 1.09 | - |
| #5 (80 mesh) | 0.13 | - |
| #6 (100 mesh) | 0.49 | 0.05 |
| #7 (120 mesh) | 0.11 | 0.43 |
| #8 (150 mesh) | 0.28 | 6.32 |
| #8 undersize fraction % | 95.18% | 99.32% |

As shown in Table 2, according to the particle size and emerging order of larger particles, Danshen extract by 2nd scheme was better than the 1st scheme in characters. Hence, the contraction way in 2nd scheme was a better option. The water extraction liquid was firstly concentrated, followed by adding with alcohol extraction liquid gradually, combined and concentrated.

Examples 1~6 are present below to provide examples of preparing Danshen extracts.

Example 1: preparation of Danshen extract

Danshen extract was prepared by a method consisting of following steps:

Danshen was refluxed with 5 times of ethanol (75%) for 2 hours and filtered to give ethanol extraction liquid and residue A left for later use;

The residue A was extracted with 5 times of water for 2 hours, filtered to give water extraction liquid and residue B to be discarded;

Respectively, the ethanol extraction liquid obtained in step (1) was stirred for 30min, cooled to 15°C or lower, allowed to stand still for 12 hours and supernatant was taken to give the ethanol supernatant; the water extraction liquid obtained in step (2) was stirred for 30min, cooled to 15°C or lower, allowed to stand still for 12 hours and supernatant was taken to give the water supernatant;

The water supernatant was concentrated to have water concentrated extraction liquid with relative density of 1.20~1.30;

The water concentrated extraction liquid was added gradually with the ethanol supernatant obtained by step (3), combined and concentrated under reduced pressure to give mixed concentrated solution with relative density ≥1.20, during concentration the relative density was kept not less than 1.10;

The mixed concentrated solution was added with 50L purified water at twice, 25L each time, well blended, concentrated under reduced pressure to relative density of 1.25-1.35 (82.5±2.5°C), filtered immediately to give Danshen extract.

Example 2: preparation of Danshen extract

Danshen extract was prepared by a method consisting of following steps:

(1) Danshen was refluxed with 2 times of ethanol (50%) for 0.5 hours and filtered to give ethanol extraction liquid and residue A left for later use;

(2) The residue A was extracted with 2 times of water for 0.5 hours, filtered to give water extraction liquid and residue B to be discarded;

(3) Respectively, the ethanol extraction liquid obtained in step (1) was stirred for 20min, cooled to 15°C or lower, allowed to stand still for 6 hours and supernatant was taken to give the ethanol supernatant; the water extraction liquid obtained in step (2) was stirred for 20min, cooled to 15°C or lower, allowed to stand still for 6 hours and supernatant was taken to give the water supernatant;

(4) The water supernatant was concentrated to have water concentrated extraction liquid with relative density of 1.20;

(5) The water concentrated extraction liquid was added gradually with the ethanol supernatant obtained by step (3), combined, concentrated under reduced pressure to give mixed concentrated solution with relative density $\geq 1.20$, during concentration the relative density was kept not less than 1.10;

(6) The mixed concentrated solution was added with 10L purified water at twice, 5L each time, well blended, concentrated under reduced pressure to relative density of 1.25 ($82.5 \pm 2.5$°C), filtered immediately to give Danshen extract.

Example 3: preparation of Danshen extract

Danshen extract was prepared by a method consisting of following steps:

(1) Danshen was refluxed with 7 times of ethanol (100%) for about 4 hours and filtered to give ethanol extraction liquid and residue A left for later use;

(2) The residue A was extracted with 7 times of water for about 4 hours, filtered to give water extraction liquid and residue B to be discarded;

(3) Respectively, the ethanol extraction liquid obtained in step (1) was stirred for 60min, cooled to 15°C or lower, allowed to stand still for 24 hours and supernatant was taken to give the ethanol supernatant; the water extraction liquid obtained in step (2) was stirred for 60min, cooled to 15°C or lower, allowed to stand still for 24 hours and supernatant was taken to give the water supernatant;

(4) The water supernatant was concentrated to have water concentrated extraction liquid with relative density of 1.30;

(5) The water concentrated extraction liquid was added gradually with the ethanol supernatant obtained by step (3), combined, concentrated under reduced pressure to give mixed concentrated solution with relative density $\geq 1.20$, during concentration the relative density was kept not less than 1.10;

(6) The mixed concentrated solution was added with 100L purified water, 50L each time, well blended, concentrated under reduced pressure to relative density of 1.35 ($82.5 \pm 2.5$°C), filtered immediately to give Danshen extract.

Example 4: preparation of Danshen extract

Danshen extract was prepared by a method consisting of following steps:

(1) Danshen was refluxed with 5 times of ethanol (75%) for about 2 hours and filtered to give ethanol extraction liquid and residue A left for later use;

(2) The residue A was extracted with 5 times of water for about 2 hours, filtered to give water extraction liquid and residue B to be discarded;

(3) Respectively, the ethanol extraction liquid obtained in step (1) was stirred for 30min, cooled to 15°C or lower, allowed to stand still for 12 hours and supernatant was taken to give the ethanol supernatant; the water extraction liquid obtained in step (2) was stirred for 30min, cooled to 15°C or lower, allowed to stand still for 12 hours and supernatant was taken to give the water supernatant;

(4) The water supernatant was concentrated to have water concentrated extraction liquid with relative density of 1.20~1.30;

(5) The water concentrated extraction liquid was added gradually with the ethanol supernatant obtained by step (3), combined, concentrated under reduced pressure to give mixed concentrated solution with relative density ≥1.20;

(6) The mixed concentrated solution was added with 50L purified water at twice, 25L each time, well blended, concentrated under reduced pressure to relative density of 1.25~1.35 (82.5±2.5°C), filtered immediately to give Danshen extract.

Example 5: preparation of Danshen extract

Danshen extract was prepared by a method consisting of following steps:

(1) Danshen was refluxed with 5 times of ethanol (75%) for about 2 hours and filtered to give ethanol extraction liquid and residue A left for later use;

(2) The residue A was extracted with 5 times of water for about 2 hours, filtered to give water extraction liquid and residue B to be discarded;

(3) Respectively, the ethanol extraction liquid obtained in step (1) was stirred for 30min, cooled to 15°C or lower, allowed to stand still for 12 hours and supernatant was taken to give the ethanol supernatant; the water extraction liquid obtained in step (2) was stirred for 30min, cooled to 15°C or lower, allowed to stand still for 12 hours and supernatant was taken to give the water supernatant;

(4) The water supernatant was concentrated to have water concentrated extraction liquid with relative density of 1.20~1.30;

(5) The water concentrated extraction liquid was added gradually with the ethanol supernatant obtained by step (3), combined, concentrated under reduced pressure to give mixed concentrated solution with relative density ≥1.20, during concentration the relative density was kept not less than 1.10;

(6) The mixed concentrated solution was added with 50L purified water at twice, 25L each time, well blended, concentrated under reduced pressure to relative density of 1.25~1.35 (82.5±2.5°C), filtered immediately to give Danshen extract.

Example 6: preparation of Danshen extract

Danshen extract: Danshen was cut. The appearance of raw medicine was reviewed and weighed for later use.
Sequence of inputting materials: Danshen was cut, inputted and decocted with 90±0.5% ethanol for the first time and first-pass RO water for second time as extraction solvent.

Extracting:

One batch of product included two extracting tanks. According to the formula, 100kg of cut Danshen were respectively weighed, placed in each tank, decocted with 400±12L ethanol (90±0.5%) for 90±5min and filtered through mesh-200 sieve to give extraction liquids. The liquids of two tanks were combined and placed in a separation tank. Resultant residue was extracted with 500±15L water for 60±3min for second time, filtered through mesh-200 sieve and the residue discarded. Water extraction liquids in two tanks were combined and placed in two different separation tanks.

Cooling and standing still:

Danshen ethanol and water mixed solutions were placed into different separation tanks, which was cooled by passing frozen water and allowed to stand still to give the extraction liquid. Resultant extraction liquid was stirred for 30min, until temperature was decreased to 15°C or lower, allowed to stand still for 6~24hours. Danshen ethanol and water extraction supernatants were respectively placed in different separation tanks.

Concentration:

Danshen water extraction liquid was firstly concentrated to relative density of 1.25~1.30 (82.5±2.5°C), into which Danshen ethanol supernatant was gradually added and concentrated. During concentration, the relative density was not less than 1.15. Until relative density of Danshen mixed extraction liquid was ≥1.34, 10L purified water was added at twice, 5L (45±5°C) each time, combined, concentrated to relative density of 1.33-1.35 (82.5±2.5°C), filtered through 40-mesh sieve immediately to give Danshen extract.

Danshen extracts of Examples 1~6 were determined by the method of present invention, and results were seen in Table 3.

Table 3: determination of Danshen extracts

| Ex. | Constituents (mg/g) | | | | | | |
|-----|-----------|-----------------|-----------------|-------------------|-----------------|-----------------|-----------|
| | Danshensu | rosmarinic acid | lithospermic acid | Salvianolic acid B | cryptotanshinone | tanshinone IIA | stachyose |
| 1 | 0.5 | 0.5 | 0.5 | 5.2 | 0.5 | 1.5 | 150.5 |
| 2 | 16.7 | 15.2 | 15.5 | 140.1 | 25.4 | 50.2 | 600.1 |
| 3 | 1.2 | 1.4 | 1.7 | 10.2 | 1.1 | 2.2 | 250.4 |
| 4 | 5.03 | 4.71 | 4.19 | 35.32 | 5.67 | 9.36 | 425.9 |
| 5 | 8.2 | 8.4 | 7.8 | 139.2 | 9.6 | 20.5 | 597.5 |
| 6 | 3.1 | 3.0 | 3.1 | 27.7 | 4.0 | 6.9 | 370.4 |

Danshen extract of Example 6 was used in following Examples 7~11 to prepare the Danshen micropellet based on conditions as follows:

Raw material:

| | |
|---|---|
| Danshen extract | 32.5kg |
| Starch or PEG-6000 blank micropellet | 16.0kg |
| Opadry 85G66817 | 1.5kg |

Preparation of Danshen extract medicine liquid: Danshen extract was placed in a premixed tank, into which 0.7~0.9 times water was added, stirred for 30min or more to give the extract medicine liquid.

Drug-loading of starch blank micropellet: atomizing pressure and spray gun were adjusted to make medicine liquid atomized into fine droplets.

Example 7: preparation of drug-loading micropellet

Feeding and fluidizing: the blank micropellet was absorbed into fluidized drying coating machine with air volume at 600m$^3$/h to have the blank micropellet fluidized completely in fluidized-bed; however, the fluidization was not too severe, so as to prevent the blank micropellet from abrading and dusting.

Drug-loading in early stage: spray gun was adjusted to make it capable of good atomizing. The extraction liquid was delivered by metering pump to the spray gun. Respectively, the pressures of upper and lower air-jet were set to 2.0Bar and 2.5Bar. Temperature of material was at about 50°C. Until temperature rose to 45°C, the feed solution began to spray at spraying rate of 70g/min.

Drug-loading stage: when the material was at 50°C, with increase of diameter of the micropellet, the spray volume was gradually increased, but the maximum rate was not more than 400g/min. Air volume was adjusted in accordance with fluidized state of micropellet.

Coating: after drug loading, coating liquid was directly sprayed to perform coating. Initially, the material temperature was set at 50°C and spraying rate 40g/min. After 20min of coating, the spraying rate was adjusted to 80g/min, material temperature controlled at 40°C. Infusion rate, air volume and material temperature were adjusted in accordance with adhesion of micropellet.

Wherein, the extraction medicine liquid was prepared by Danshen extract and water in a weight ratio of 100:90.

Example 8: preparation of drug-loading micropellet

Feeding and fluidizing: the blank micropellet was absorbed into fluidized drying coating machine with air volume at 1500m$^3$/h to have the blank micropellet fluidized completely in fluidized-bed;

Drug-loading in early stage: spray gun was adjusted to make it capable of good atomizing. The extraction liquid was delivered by metering pump to the spray gun. Respectively, the pressures of upper and lower air-jet were set to 3.0Bar and 3.5Bar. Temperature of material was at about 50°C. Until temperature rose to 45°C, the feed solution began to spray at spraying rate of 120g/min.

Drug-loading stage: when the material was at 55°C, with increase of diameter of the micropellet, the spray volume was gradually increased, but the maximum rate not more than 400g/min. Air volume was adjusted in accordance with fluidized state of micropellet.

Coating: after drug loading, coating liquid was directly sprayed to perform coating. Initially, the material temperature was set at 50°C and spraying rate 80g/min. After 20min of coating, the spraying rate was adjusted to 150g/min, material temperature controlled at 55°C. Infusion rate, air volume and material temperature were adjusted in accordance with adhesion of micropellet.

Wherein, the extraction medicine liquid was prepared by Danshen extract and water in a weight ratio of 100:70.

Example 9: preparation of drug-loading micropellet

Feeding and fluidizing: the blank micropellet was absorbed into fluidized drying coating machine with air volume at 1500m$^3$/h to have the blank micropellet fluidized completely in fluidized-bed;

Drug-loading in early stage: spray gun was adjusted to make it capable of good atomizing. The extraction liquid was delivered by metering pump to the spray gun. Respectively, the pressures of upper and lower air-jet were set at 3.0Bar and 3.5Bar. Temperature of material was at about 50°C. Until temperature rose to 45°C, the feed solution began to spray at spraying rate of 120g/min.

Drug-loading stage: when the material was at 55°C, with increase of diameter of the micropellet, the spray volume was gradually increased, but the maximum rate not more than 400g/min. Air volume was adjusted in accordance with fluidized state of micropellet.

Coating: after drug loading, coating liquid was directly sprayed to perform coating. Initially, the material temperature was set at 50°C and spraying rate 80g/min. After 20min of coating, the spraying rate was adjusted to 150g/min, material temperature controlled at 55°C. Infusion rate, air volume and material temperature were adjusted in accordance with adhesion of micropellet.

Wherein, Danshen extract as the extract and the starch micropellet as the blank micropellet were in a weight ratio of 2:1; the medicine liquid was prepared by Danshen extract and water in a weight ratio of 100:82.

Example 10: preparation of drug-loading micropellet

Feeding and fluidizing: the blank micropellet was absorbed into fluidized drying coating machine with air volume at 1500m$^3$/h to have the blank micropellet fluidized completely in fluidized-bed; however, the fluidization was not too severe, so as to prevent the blank micropellet from abrading and dusting.

Drug-loading in early stage: 0.2mm spacer was used in spray gun that was adjusted to make it capable of good atomizing. The extraction liquid was delivered by metering pump to the spray gun. Respectively, the pressures of upper and lower air-jet were set at 3.0Bar and 3.5Bar. Temperature of material was at about 50°C. Until temperature rose to 45°C, the feed solution began to spray at spraying rate of 120g/min.

Drug-loading stage: when the material was at 55°C, with increase of diameter of the micropellet, the spray volume was gradually increased, but the maximum rate not more than 400g/min. Air volume was adjusted in accordance with fluidized state of micropellet.

Coating: after drug loading, coating liquid was directly sprayed to perform coating. Initially, the material temperature was set at 50°C and spraying rate 80g/min. After 30min of coating, the spraying rate was adjusted to 150g/min, material temperature controlled at 55°C. Infusion rate, air volume and material temperature were adjusted in accordance with adhesion of micropellet.

Wherein, Danshen extract as the extract and sucrose micropellet as the blank micropellet were in a weight ratio of 1:1; the medicine liquid was prepared by Danshen extract and water in a weight ratio of 100:90.

Example 11: preparation of Danshen micropellet capsule

Preparation of Danshen extract medicine liquid: Danshen extract was placed in premixed tank, into which 0.82 times purified water was added, stirred for 30min or more to give the medicine liquid;

Drug-loading of starch blank micropellet: spray gun was adjusted to make it capable of good atomizing;

Feeding and fluidizing: the starch blank micropellet was absorbed into fluidized drying coating machine with air volume at 1000m³/h to have the blank micropellet fluidized completely in fluidized-bed; however, the fluidization was not too severe, so as to prevent the blank micropellet from abrading and dusting. The premixed extraction liquid was delivered by metering pump to the spray gun. Respectively, the pressures of upper and lower air-jet were set at 2.5Bar and 3Bar. In the early stage of drug-loading, temperature of material was at about 50°C. Until temperature rose to 45°C, the feed solution began to spray at spraying rate of 120g/min.

Drug-loading stage: when the material was at 50°C, with increase of diameter of the micropellet, the spray volume was gradually increased, but the maximum rate not more than 400g/min. Air volume was adjusted in accordance with fluidized state of micropellet. During drug-loading process, samples should be taken from sampling port, if necessary, to observe adhesion and dusting of micropellet. Depending on the observation, infusion rate, air volume and material temperature were adjusted.

Coating: opadry 85G66817 and purified water were stirred uniformly for not less than 45min to give 18% coating liquid for later use. After drug loading, coating liquid was directly sprayed to perform coating. Initially, the material temperature was set to 50°C and spraying rate 80g/min. After 20min of coating, the spraying rate was adjusted to 100g/min, material temperature controlled at 50°C. Infusion rate, air volume and material temperature were adjusted in accordance with adhesion of micropellet. After coating, temperature was decreased to 35°C to discharge the material.

Screening of coated micropellets: intermediate coated micropellets were screened, and the inner diameter of mesh 1.8mm. The undersized was Danshen micropellet, accounting for not less than 90%.

Loading capsule: the screened Danshen micropellet was loaded into capsule with capsule loading machine to give the capsule.

Bulk density, specific surface area and particle size of the micropellet obtained from Examples 7~11 had been determined by a method as follows:

The particle size was assayed by a conventional method, e.g. the pharmacopoeia sieve of 7# provision under measurement item in legend of Chinese Pharmacopoeia (I). Sieving method was used to assay particle size.

Bulk density was assayed by conventional viscosity measurement method, e.g. BT-1000 comprehensive powder characteristic testing instrument (developed jointly by Dandong Better Apparatus Inc. and Qinghua University). The bulk density of particle was assayed by instrument requirements.

Specific surface area was assayed by a conventional method, e.g. the specific surface area analyzer SSA-3600 (Beijing Biaode Electronic Technology Inc.). The specific surface area of micropellet was assayed by instrument requirements.

The results involved were seen in Table 4.

Table 4: bulk density, specific surface area and particle size of micropellet

| Example | Bulk density (g/ml) | Specific surface area (m²/g) | Particle size (mm) |
|---------|---------------------|------------------------------|--------------------|
| 7 | 1.02 | 0.02 | 1.01 |
| 8 | 0.62 | 0.005 | 1.83 |
| 9 | 1.25 | 0.05 | 0.48 |
| 10 | 0.81 | 0.01 | 1.22 |
| 11 | 1.09 | 0.03 | 0.69 |

Effect of Danshen extract in present invention was demonstrated in following efficacy trial.

Trial example 1: effect of Danshen extract on auricle microcirculation in mice

In order to evaluate effect of Danshen extract on microcirculation, the trial was carried out by comparing NA-caused auricle microcirculation disorder model and normal mice to evaluate the effect of Danshen extract on microcirculation. Tested drug was divided into three dose groups of 7, 14, 28g raw medicine/kg (2, 4, 8g extract/kg), which were administrated intragastrically for 7 days. Vascular caliber and flow rate of auricle microcirculation in mice was assayed with microcirculation measuring instrument after last administration.

1. Materials

1.1 Tested drug

Danshen extract prepared by the method of Example 6, dark brown extract (1g extract corresponds to 3.5g raw medicine)

1.2 Positive drug

Compound Danshen dripping pill (CDDP), 27mg/pill, provided by Tasly Pharmaceutical Group Co., Ltd.

1.3 Animal

1.3.1 Species, specification and source

ICR mice of clean grade, weighing 18~22g, half male and female, were purchased from comparative medicine center of Yangzhou University, and certification No.: SCXK 2012-0004.

1.3.2 Feeding condition

Mice were raised in independent ventilation cage (IVC) with air cleanliness of 10000. Temperature of laboratory was 24±2°C, humidity of 60%-80% and air changed 10~15 times/hour. Lighting period: 12 (L)/12 (D) hour. Male and female mice were raised separately, not more than 5 each cage.

Feed: Full value nutrient feed for mice was purchased Jiangsu Xietong Medical Bio-engineering Inc. Quality was in line with the standard GB14924.1-2001 (Laboratory animals General quality standard for formula feeds).

Padding: sterilized particle padding was purchased from Jiangsu Xietong Medical Bio-engineering Inc.

Water: drinkable purified water

1.4 Reagent

Adrenaline hydrochloride injection, 1ml:1mg, batch No.: 120915 Shanghai Hefeng Pharmaceutical Inc.

Urethane, 500g/bottle, analytically pure, purchased from Shanghai Qingxi Chemical Technology Inc.

1.5 Apparatus

BSA124S precision electronic balance (0.1mg-120g), Sartorius, German
KD-160 electronic scale, Dongguan Bailida Health Equipment Inc.

WXT-4 colorful multi-site circulating indicator, Xuzhou Hengda Optical Electronic Inc.

2 Method

2.1 Dose setting

According to the previous research, on the basis of preliminary experiment, three dose groups were set in tested drugs: 7, 14, 28 raw medicine/kg (2, 4, 8g extract/kg). Clinical dose of Danshen extract was 10g raw medicine /person/day, equivalent dose calculated for mice was 2g raw medicine /kg. Hence, three doses in this research were respectively 3.5, 7 and 14 times of clinical equivalent dose.

According to related literature and historic result, one dose group was set in positive drug (CDDP): 270mg (10 pills)/kg.

2.2 Effect on auricle microcirculation in normal mice

50 mice were randomly divided into 5 groups, 10 mice each groups: blank control group, Danshen extract group (2, 4, 8g extract/kg) and CDDP group (270mg/kg). Each group was administrated intragastrically respectively and the blank control group was given with distilled water of same volume, once a day. On 6th day after administration, all mice were fasted overnight with free access to water. After 7 days of administration, urethane (20%, 0.1 ml/10g) was injected intraperitoneally to provide anaesthesia. Auricle hair was plucked out with medical adhesive plaster, and mice were placed in a lateral position on the observation board. After adding liquid paraffin dropwise, by a small glass rod, the ear was flattened on the ear holder with back up. Auricle veins and capillaries in the same site were selected to observe the change of vascular caliber and flow rate of auricle microcirculation in mice with microcirculation measuring instrument respectively before administration and at 0.5, 2 and 4 hours after administration.

2.3 Effect on NA-caused microcirculation disturbance

60 mice were randomly divided into 6 groups, 10 mice each group: blank control group, model group, Danshen extract groups (2, 4, 8g extract/kg) and CDDP group (270mg/kg). Blank control group and model group were administrated with distilled water of same volume, once a day. On 6th day after administration, all mice were fasted overnight with free access to water.

After 7 days of administration, urethane (20%, 0.1ml/10g) was injected intraperitoneally to provide anaesthesia. Auricle hair was plucked out with medical adhesive plaster, and mice were placed in a lateral position on the observation board. After adding liquid paraffin dropwise, by a small glass rod, the ear was flattened on the ear holder with back up. Auricle veins and capillaries in the same site were selected and assayed with microcirculation measuring instrument. 30min after last intragastric administration, 5 groups, except blank control group, were immediately injected with NA (100μg/kg) via the tail veins. The change of vascular caliber and flow rate of auricle microcirculation in mice were observed respectively before administration and at 5, 30 and 120 min after administration.

2.4 Statistical method

Data were expressed as "M±SD", and t-test was used to compare between groups.

3. Results

3.1 Effect on auricle microcirculation in normal mice

Compared with the blank control group at the same time point, diameter in input part and output part at each time point in high-dose Danshen extract group were increased significantly (P<0.05, P<0.01). In middle-dose Danshen extract group, diameter in input part and output part at each time point were increased significantly (P<0.05, P<0.01) within 2 hours after administration. In low-dose Danshen extract group, diameter in input part and output part at each time point were increased markedly, having statistical significance, compared with the blank control group (Tables 5 and 6).

At the same time point, compared with the blank control group, blood flow rate of auricle microcirculation was increased significantly within 0.5 hours after administration in each dose Danshen extract group, having statistical significance (Table 7).

Table 5: effect of Danshen extract on diameter of input part of auricle microcirculation in normal mice (N=10, M±SD)

| Groups | dose (g/kg) | Diameter of input part (μm) | | | |
|---|---|---|---|---|---|
| | | 0h | 0.5h | 2h | 4h |
| Blank control group | | 21.3±6.9 | 23.1±3.4 | 26.4±5.8 | 26.8±8.0 |
| CDDP group | 0.27 | 23.5±5.3 | 30.9±9.5* | 31.6±7.7* | 27.8±9.8 |
| Low-dose Danshen extract | 2 | 21.3±9.1 | 27.1±5.2* | 29.9±5.8 | 29.5±7.1 |
| middle-dose Danshen extract | 4 | 22.0±8.1 | 29.2±7.3* | 30.2±7.4* | 29.1±5.6 |
| High-dose Danshen extract | 8 | 22.0±4.1 | 33.7±11.0* | 35.4±9.4* | 27.4±4.0 |
| *P<0.05, **P<0.01, compared with the blank control group. | | | | | |

Table 6: effect of Danshen extract on diameter of output part of auricle microcirculation in normal mice (N=10, M±SD)

| Groups | Dose (g/kg) | Diameter of output part (µm) | | | |
|---|---|---|---|---|---|
| | | 0h | 0.5h | 2h | 4h |
| Blank control group | | 40.1±14.9 | 50.2±22.3 | 51.4±14.8 | 49.8±20.6 |
| CDDP group | 0.27 | 58.7±14.6* | 88.9±20.5** | 74.2±17.5** | 56.5±17.8 |
| Low-dose Danshen extract | 2 | 49.0±16.1 | 66.4±18.5* | 60.8±20.9 | 56.9±13.3 |
| middle-dose Danshen extract | 4 | 55.8±20.0 | 74.8±21.0* | 73.7±21.6* | 62.0±21.9 |
| High-dose Danshen extract | 8 | 76.0±28.4** | 99.6±32.8** | 87.9±29.9** | 81.3±21.4** |
| *P<0.05, **P<0.01, compared with the blank control group. | | | | | |

Table 7: effect of Danshen extract on blood flow rate of auricle microcirculation in normal mice (N=10, M±SD)

| Groups | Dose (g/kg) | Flow rate (µm/s) | | | |
|---|---|---|---|---|---|
| | | 0h | 0.5h | 2h | 4h |
| Blank control group | | 588.0±43.6 | 573.6±33.5 | 552.6±45.1 | 588.4±31.3 |
| CDDP group | 0.27 | 590.6±44.0 | 619.7±32.3* | 609.1±45.2 | 604.1±38.3 |
| Low-dose Danshen extract | 2 | 580.0±33.6 | 613.6±39.5* | 562.6±75.1 | 588.4±51.3 |
| middle-dose Danshen extract | 4 | 590.0±24.4 | 637.3±52.2* | 570.8±76.5 | 596.8±71.8 |
| High-dose Danshen extract | 8 | 583.1±36.7 | 639.0±48.5* | 611.8±55.5* | 599.1±52.4 |
| *P<0.05, compare with the blank control group. | | | | | |

3.2 Effect on NA-caused microcirculation disturbance

Compared with the blank control group, within 30min after making model with NA, diameters in input part and output part of auricle microcirculation in mice were deceased significantly (P<0.05, P<0.01) and blood flow rate reduced partially. At $120^{th}$ min after making model, changes of microvascular had gradually disappeared.

Danshen extract in each dose group had improving effect on NA-caused lower diameter in input part and output part in different levels (P<0.05, P<0.01) and a certain effect on increasing blood flow rate (Tables 8, 9, 10).

Table 8: effect of Danshen extract on diameter of input part of auricle microcirculation in NA-caused mice (N=10, M±SD)

| Groups | Dose (g/kg) | Diameter of input part (µm) | | | |
|---|---|---|---|---|---|
| | | Omin | 5min | 30min | 120min |
| Blank control group | | 27.6±7.5 | 28.4±4.7 | 27.4±7.0 | 27.9±5.5 |
| Model group | | 30.1±7.9 | 20.0±5.1* | 19.3±3.6** | 24.8±5.1 |
| CDDP group | 0.27 | 31.4±7.4 | 28.0±6.5# | 29.3±10.3# | 28.6±7.2 |
| Low-dose Danshen extract | 2 | 29.3±5.6 | 28.6±5.4# | 23.1±4.1 | 24.0±10.2 |

(continued)

| Groups | Dose (g/kg) | Diameter of input part ($\mu$m) | | | |
|---|---|---|---|---|---|
| | | 0min | 5min | 30min | 120min |
| middle-dose Danshen extract | 4 | 35.4±9.8 | 28.5±7.3# | 24.9±5.3# | 24.0±6.3 |
| High-dose Danshen extract | 8 | 31.8±10.7 | 27.0±8.2# | 27.7±5.9## | 26.9±4.9 |
| *P<0.05, **P<0.01, compared with the blank control group ; #P<0.05, ##P<0.01, compared with the model control group. | | | | | |

Table 9: effect of Danshen extract on diameter of output part of auricle microcirculation in NA-caused mice (N=10, M±SD)

| Groups | Dose (g/kg) | Diameter of output part ($\mu$m) | | | |
|---|---|---|---|---|---|
| | | 0min | 5min | 30min | 120min |
| Blank control group | | 52.0±20.9 | 52.3±13.5 | 51.6±11.8 | 47.9±14.9 |
| Model group | | 53.7±13.2 | 40.4±8.3** | 38.0±11.9* | 46.8±11.8 |
| CDDP group | 0.27 | 56.5±20.9 | 50.9±15.8# | 51.7±15.0# | 50.1±8.6 |
| Low-dose Danshen extract | 2 | 58.4±17.1 | 50.1±14.7# | 47.1±10.9# | 49.6±19.2 |
| middle-dose Danshen extract | 4 | 56.8±11.1 | 54.5±10.9# | 54.9±12.2# | 52.9±21.9 |
| High-dose Danshen extract | 8 | 59.8±19.5 | 61.9±23.5# | 55.5±15.0# | 51.1±12.6 |
| *P<0.05, **P<0.01, compared with the blank control group; #P<0.05, compared with the model control group. | | | | | |

Table 10: effect of Danshen extract on blood flow rate of auricle microcirculation in NA-caused mice (N=10, M±SD)

| Groups | Dose (g/kg) | Flow rate ($\mu$m/s) | | | |
|---|---|---|---|---|---|
| | | 0min | 5min | 30min | 120min |
| Blank control group | | 389.3±147.5 | 390.6±135.7 | 399.4±108.4 | 379.2±111.5 |
| Model group | | 397.5±81.1 | 312.8±90.8 | 354.3±178. 9 | 390.9±183.2 |
| CDDP group | 0.27 | 367.1±143.2 | 420.2±131.9# | 402.4±132.0 | 400.9±176.0 |
| Low-dose Danshen extract | 2 | 354.4±38.3 | 378.8±42.4 | 367.6±17.7 | 388.9±48.5 |
| middle-dose Danshen extract | 4 | 350.8±81.8 | 387.7±91.6 | 369.0±160.8 | 439.6±104.9# |
| High-dose Danshen extract | 8 | 408.5±145.3 | 420.1±134.4# | 397.9±193.7 | 399.9±113.7 |
| #P<0.05, compared with the model group. | | | | | |

As shown in this trial example, Danshen extract was confirmed to have obviously increasing effect on diameter in input part and output part in normal mice, and a certain effect on increasing blood flow rate of microvascular of auricle microcirculation.

Compared with normal mice, within 30min after making model with NA, diameter in input part and output part of auricle microcirculation in mice were significantly decreased (P<0.05, P<0.01), blood flow rate decreased partially.

At 120th min after making model, changes of microvascular had gradually disappeared. Danshen extract in each dose group had improving effect on NA-caused lower diameter in input part and output part in different levels (P<0.05, P<0.01) and little effect on blood flow rate.

In summary, Danshen extract of present invention could obviously enhance the angiectasia of the capillaries of auricle microcirculation and further improve the microcirculation.

Trial example 2: effect of Danshen extract on reducing blood lipid in zebra fish model

1. Reagent and apparatus

Tested drug: Danshen extract, prepared by the method of Example 6, was diluted with ultrapure water to prepare into stock solution, preserved at -20°C.

Apparatus and reagent: dissecting microscope (SZX7, Olympus Inc), 6 orifice plate (Nest Biotech), MESAB (Sigma), Methyl cellulose (Sigma), lovastatin (Meilun Bio-tech Inc, Dalian, Batch No.: 20111002, purity>99%, powdery), high-fat food (Huante Inc, Hangzhou), Oil red (Sigma, batch No.: 20120411).

Animal: Zebra fish embryos were propagated by means of natural pair mating. Each mating, 4~5 pairs of adult were prepared, and in average, each pair of zebra fish produced 200~300 embryos. At 6th hour post-fertilization (6hpf) and 24hpf, the embryos were cleaned (removing dead embryo) and qualified embryos were selected according to embryo developing stage (Kimmel et al. 1995). At 28°C, embryos were incubated in fish-farming water, which contained 200mg instant soluble sea salt in 1L reverse osmosis water with conductivity of 480-510 $\mu$S/cm, pH of 6.9-7.2, hardness of 53.7-71.6 mg/L $CaCO_3$. Feeding was required since 9 days post-fertilization (9dPf) because the embryos could get nutrients from their own yolk sac. After study, tricaine methane sulfonate (TMS) was used to overexpose the embryos in various developmental stages and the zebra fish were anesthetized and sacrificed. Sacrifice procedure was in light of the sacrifice-under-anesthesia regulatory requirement of AVMA (America Veterinary medical Association).

2. Method

A. Determination of maximum non-lethal concentration (MNLC) of Danshen extract

High-fat food was used to feed juvenile fish of zebra fish of ALBINO strain to induce zebra fish high blood lipid model.

After feeding, the zebra fish high blood lipid model was treated with Danshen extract. Several concentrations were set and 30 zebra fish treated in each concentration.

Five initial concentrations of Danshen extract were respectively 200$\mu$g/ml, 400$\mu$g/ml, 600$\mu$g/ml, 800$\mu$g/ml, 1200$\mu$g/ml and 2000$\mu$g/ml (prepared with purified water).

48 hours after treatment, the zebra fish number of dearth was counted and the optimum concentration-effect curve was drawn with GraPhPad Prism5.0 statistical software to calculate MNLC.

B. Quantitative evaluation of blood lipid lowering effect of Danshen extract

According to results of part A, the blood lipid lowering effect of Danshen extract in three concentrations was evaluated (usually at MNLC, 1/3MNLC and 1/10 MNLC), and 30 zebra fish treated in each concentration.

High-fat food was used to feed juvenile fish of zebra fish of ALBINO strain to induce zebra fish high blood lipid model.

After feeding, the zebra fish high blood lipid model was treated with tested drug for 48 hours.

Positive drug: lovastatin at 0.08 $\mu$g/ml.

After being treated with tested drugs, zebra fish were fat stained with fat-specific colorant.

Fifteen zebra fish were randomly selected in each concentration to observe intensity of zebra fish tail blood fat stain, pictured and saved.

Image -Pro Plus 6.0 image analytical software was used to analyze the image. The signal strength of zebra fish tail blood fat stain (S) was calculated and quantitatively analyzed. The statistical results were expressed as $\overline{X} \pm$ SE.

The calculation formula of blood lipid lowing efficacy of Danshen extract was presented as follows:

$$\text{Blood lipid reducing rate (\%)} = \frac{[1 - \frac{S(medicine)}{S(control)}]}{} \times 100\% \qquad (1)$$

Variance analysis and Dunnett's t-test were used for statistical analysis, P<0.05 showed significant difference.

3. Results

3.1 Determination of MNLC of Danshen extract

Zebra fish dearth rate induced by Danshen extract was seen in Table 11.

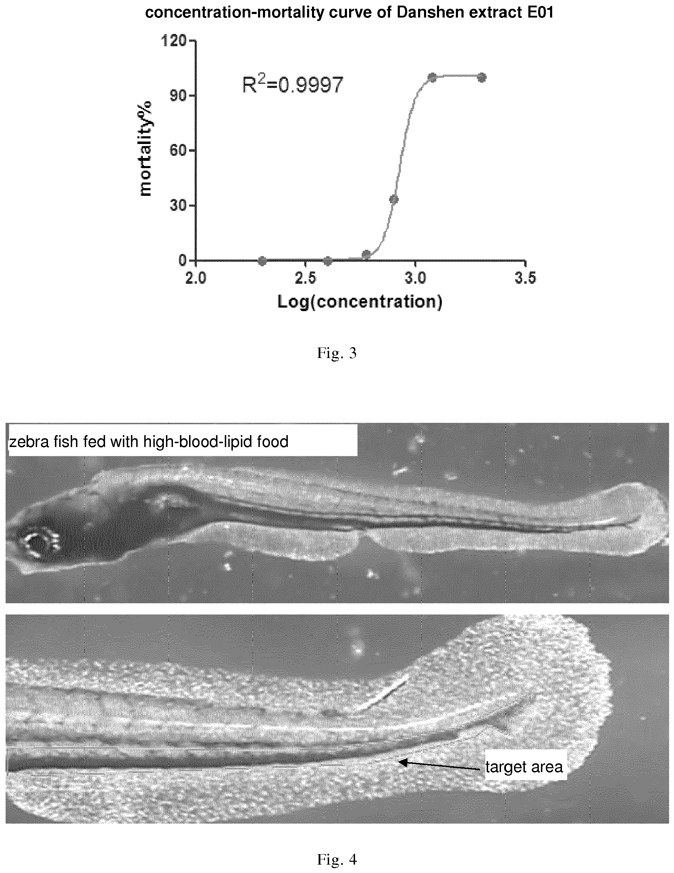

As shown in Table 11, GraPhPad 5.0 was used to fit lethal-concentration curve (Fig.3). After fitting, Danshen extract MNLC was calculated as 540μg/ml (extract) corresponding to 1890μg/ml (raw medicine).

Table 11: zebra fish dearth rate induced by Danshen extract (n=30)

| Extract concentration (μg/m 1) | Corresponding concentration of raw medicine (μg/ml) | Death rate (%) |
|---|---|---|
| 0 | 0 | 0 |
| 200 | 700 | 0 |
| 400 | 1400 | 0 |
| 600 | 2100 | 3.3 |
| 800 | 2800 | 33.3 |
| 1200 | 4200 | 100 |
| 2000 | 7000 | 100 |

3.2 Quantitative evaluation or blood lipid lowering effect of Danshen extract

According to the lethal experiment, the blood lipid lowering effect of Danshen extract was evaluated in three concentrations (calculated as raw medine): 1890 μg/ml (MNLC), 630μg/ml (1/3MNLC) and 189 μg/ml (1/10MNLC).

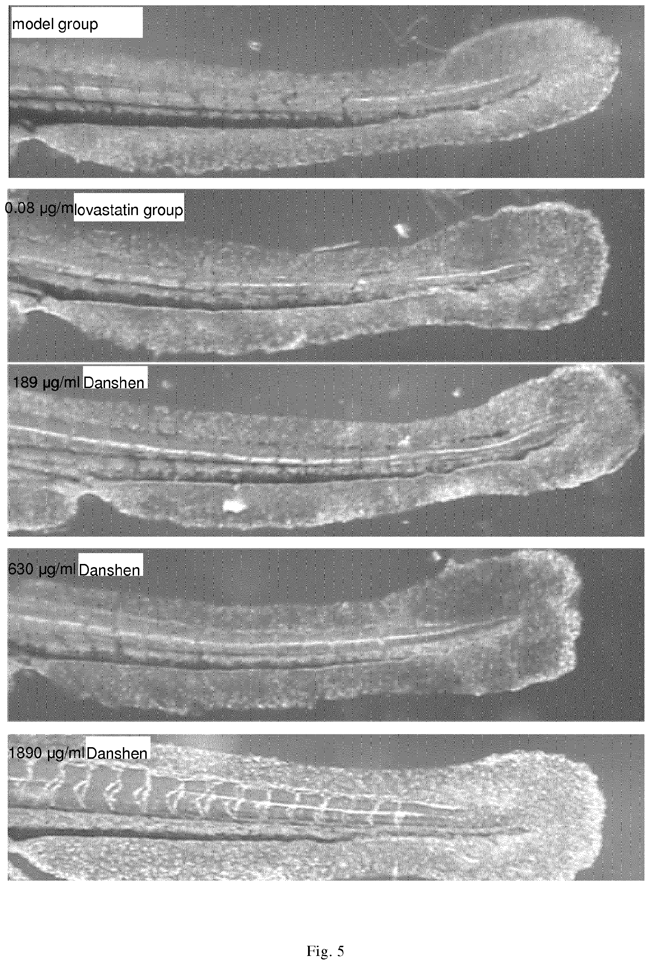

After being treated with Danshen extract, zebra fish were fat-specific stained, observed under microscope, pictured and preserved (Fig.4). As shown in Fig.4, solid line circled area was observed as target area. Strength of fat staining was quantitatively analyzed with image analytical software to calculate IOD (integral optical density) of zebra fish (seen in Table 12). According to IOD, blood lipid reducing rate of zebra fish was calculated with Formula (1) (seen in Table 12 and Fig.5) to evaluate efficacy of Danshen extract on reducing blood lipid. Strength of zebra fish tail fat staining in the positive group (lovastatin 0.08 μg/ml) was much weaker than the model group, reducing rate 26.3% (P<0.01). At 189 μg/ml, 630 μg/ml and 1890 μg/ml, Danshen extract significantly reduced the strength of zebra fish tail vascular fat staining (P<0.001, P<0.001, P<0.001), reducing rate 32.7%, 35.2% and 36.2%, respectively.

Table 12: effect on reducing blood lipid 48 hours after treated with Danshen extract (Mean±SE)

| Groups | Concentration of raw medicine (μg/ml) | Tail blood lipid IOD | Blood lipid reducing rate % |
|---|---|---|---|
| Model group | - | 2906±131 | - |
| Lovastatin group | 0.081 | 2142±109* | 26.3* |
| Danshen extract | 189 | 1955±149** | 32.7** |
| | 630 | 1884±158** | 35.2** |
| | 1890 | 1854±211** | 36.2** |
| Compared with model group, *: P<0.01, **: P<0.001 | | | |

As shown in this trial example, at 189 μg/ml, 630 μg/ml and 1890 μg/ml, Danshen extract significantly reduced strength of tail vascular fat staining, blood lipid reducing rate 32.7% (P<0.001), 35.2% (P<0.001) and 36.2% (P<0.001), respectively. It was indicated that Danshen extract had effect of reducing blood lipid, and the efficacy approached a plateau phase at 1/3 MNLC.

**Claims**

1. A Danshen (salvia miltiorrhiza) extract, **characterized in that** said extract comprises following components by weight part: Danshensu : rosmarinic acid : lithospermic acid : salvianolic acid B : cryptotanshinone : tanshinone IIA : stachyose=(0.5-16) : (0.5-15) : (0.5-15) : (5-140) : (0.5-25) : (1-50) : (150-600); preferably said components by weight part: (1-8) : (1-8) : (1-8) : (10-70) : (1-10) : (2-20) : (250-500); more preferably said components by weight part: (2-5) : (2-5) : (2-5) : (25-60) : (2-6) : (4-10) : (300-450); further more preferably said components by weight part: (2-4) : (2-4) : (2-4) : (25-30) : (2-5) : (4-10) : (330-400); most preferably said components by weight part: 3 : 3 : 3 : 28 : 4 : 7 : 370.

2. A micropellet formulation comprising said Danshen extract of claim 1 as active ingredient; preferably the bulk density of said micropellet is 0.6~1.3g/ml, specific surface area 0.005-0.05 $m^2$/g and particle size 0.5-1.8mm; more preferably the bulk density of said micropellet is 0.8~1.1g/ml, specific surface area 0.01-0.03 $m^2$/g and particle size 0.7-1.2mm.

3. A micropellet capsulate formulation prepared from the micropellet formulation of claim 2.

4. A method of preparing Danshen extract as defined in claim 1 which comprises steps as follows:

   (1) Danshen is extracted with alcohol and filtered to give alcohol extraction liquid and residue A for later use; preferably said alcohol is ethanol at concentration of 50~100% (v/v) in amount of 2~7 times the weight of crude medicine and extraction time 0.5~4 hours;
   (2) the residue A is extracted with water, filtered to give water extraction liquid and residue B to be discarded; preferably the amount of water added is 3~7 times the weight of the residue A and extraction time 0.5~4 hours;
   (3) respectively, the alcohol and water extraction liquids are cooled and allowed to stand still and both supernatants are taken out to give the alcohol and water supernatant respectively; preferably said cooling and standing still includes steps as follows: the extraction liquid is stirred for 20~60 min and allowed to stand still for 6~24 hours with the temperature decreased to 15°C or lower, before the supernatant is taken out;
   (4) the water supernatant is concentrated to have water concentrated extraction liquid; preferably the water supernatant is concentrated to relative density of 1.10~1.35 to give the water concentrated extraction liquid;
   (5) the water concentrated extraction liquid is added with the alcohol supernatant gradually, combined and concentrated to give mixed concentrated solution;
   (6) the mixed concentrated solution is added with purified water, well blended to give Danshen extract; preferably the mixed concentrated solution is added with 10L~100L purified water for several times, 5L~50L each time, blended and concentrated under reduced pressure to give the extract with relative density of 1.25~1.35 at 82.5±2.5°C.

5. The method of claim 4, **characterized in that** said method comprises steps as follows:
   Ground Danshen is taken, added with 400±12L ethanol (90±0.5%), decocted for 90±5min, filtered through 200-mesh sieve, blended and put into separation tank; resultant residue is added with 500±15L water for 2nd decoction of 60±3min, filtered through 200-mesh sieve and the residue is discarded; the water extraction liquid is blended and put into different tanks; alcohol mixed extraction liquid and water mixed extraction liquid are placed in different separation tanks, which are passed with frozen water to cool and allowed to stand still; after 30 min of stirring, the extraction liquid is cooled to 15°C or lower within 4 hours and allowed to stand still for 6~24 hours; water supernatant and alcohol supernatant are transferred to respective tanks; firstly, the water supernatant is concentrated to water concentrated extraction liquid with relative density of 1.25~1.30 (82.5±2.5°C), into which the alcohol supernatant is added gradually and further blended and concentrated; during process of concentration, density of the concentrated solution is not less than 1.15; when the density of mixed concentrated solution is ≥1.34, 10L purified water is added at twice, 5L each time (45±5°C), blended, concentrated to relative density of 1.33~1.35 (82.5±2.5°C), and filtered immediately through 40-mesh sieve to give the Danshen extract.

6. A method of preparing micropellet containing Danshen extract as defined in claim 3 which comprises steps as follows:

   (1) Danshen extract and blank micropellet in a weight ratio of 1:5~5:1 are taken for later use;
   (2) feeding and fluidizing: the blank micropellet of formula dosage is absorbed into fluidized drying coating machine to have the blank micropellet completely fluidized in fluidized-bed;
   (3) drug-loading in early stage: until the material temperature reaches 40-60°C, a liquid is sprayed and spread on the surface of the micropellet at a rate of 70-120g/min; said liquid as sprayed is small droplets prepared by atomizing premixed Danshen extract obtained by diluting Danshen extract with water;

(4) drug-loading stage: when the material is at 40~60°C, with increase of diameter of the micropellet, the spray volume is gradually increased; diameter of micropellet and spraying rate are increased gradiently to give a granule with a diameter of 0.5~1.8mm.

7.  The method of claim 6, **characterized in that** said method additionally comprises step (5):
    after spraying, the drug-loading micropellet is coated by spraying coating liquid; temperature of coating material is 40-60°C, spraying rate 40-300g/min, coating time 1~4 hours and concentration of the coating liquid 5~25%.

8.  The method of claim 7, **characterized in that** said method comprises steps as follows:

    (1) Danshen extract and blank micropellet in a weight ratio of 1:3~3:1, preferably 2:1~1:1, are taken for later use;
    (2) feeding and fluidizing: at air volume of 600-1500m$^3$/h, the blank micropellet is absorbed into fluidized drying coating machine to have the blank micropellet completely fluidized in fluidized-bed; however, the fluidization cannot be too severe, so as to prevent the blank micropellet from abrading and dusting; due to this, the blank micropellet should be covered with the droplets atomized by spray gun;
    (3) drug-loading in early stage: spray gun and spray pressure are adjusted to make liquids atomized into small droplet; the extract is delivered to the spray gun by metering pump; the pressures of upper and lower air-jet are respectively set to 2.0~3.0Bar and 2.5~3.5Bar; temperature of material is at 50°C; until temperature reaches 45°C, feed solution begins to spray at a rate of 120g/min;
    (4) drug-loading stage: when the material is at 45~55°C, with increase of diameter of the micropellet, spray volume is gradually increased, but the maximum rate not more than 400g/min; air volume is adjusted in accordance with fluidized state of the micropellet;
    (5) coating: after drug loading, coating liquid is directly sprayed to perform coating; initially, the material temperature is set to 50°C and spraying rate 80g/min; after 20min of coating, the spraying rate can be adjusted to 80-150g/min, material temperature controlled at 40-55°C; infusion rate, air volume and material temperature are adjusted in accordance with adhesion of micropellet;

    preferably, extract medicine solution is prepared by dilution of Danshen extract with water; the weight ratio of Danshen extract to water is 100 : 60-100, preferably 100 : 70-90, more preferably 100 : 75-85.

9.  The method of any one of claims 6~8, **characterized in that** said micropellet formulation is the micropellet formulation of claim 2.

10. The Danshen extracts of claim 1 or the micropellet formulation of claim 2 or the micropellet capsulate formulation of claim 3 for use in improving microcirculation or reducing blood lipid.


**Patentansprüche**

1.  Danshen-(Salvia miltiorrhiza)-Extrakt, **dadurch gekennzeichnet, dass** der Extrakt die folgenden Komponenten in Gewichtsteilen umfasst: Danshensu : Rosmarinsäure : Lithosperminsäure: Salvianolsäure B : Cryptotanshinon : Tanshinon IIA : Stachyose = (0,5 bis 16) : (0,5 bis 15) : (0,5 bis 15) : (5 bis 140) : (0,5 bis 25) : (1 bis 50) : (150 bis 600); vorzugsweise die Komponenten in Gewichtsteilen: (1 bis 8) : (1 bis 8) : (1 bis 8) : (10 bis 70) : (1 bis 10) : (2 bis 20) : (250 bis 500); stärker bevorzugt die Komponenten in Gewichtsteilen: (2 bis 5) : (2 bis 5) : (2 bis 5) : (25 bis 60) : (2 bis 6) : (4 bis 10) : (300 bis 450); noch stärker bevorzugt die Komponenten in Gewichtsteilen: (2 bis 4) : (2 bis 4) : (2 bis 4) : (25 bis 30) : (2 bis 5) : (4 bis 10) : (330 bis 400); am stärksten bevorzugt die Komponenten in Gewichtsteilen: 3 : 3 : 3 : 28 : 4 : 7 : 370.

2.  Mikropellet-Formulierung, umfassend den Danshen-Extrakt nach Anspruch 1 als Wirkstoff; wobei die Schüttdichte des Mikropellets 0,6 bis 1,3 g/ml, die spezifische Oberfläche 0,005 bis 0,05 m$^2$/g und die Partikelgröße 0,5 bis 1,8 mm beträgt; wobei stärker bevorzugt die Schüttdichte des Mikropellets 0,8 bis 1,1 g/ml, die spezifische Oberfläche 0,01 bis 0,03 m$^2$/g und die Partikelgröße 0,7 bis 1,2 mm beträgt.

3.  Eingekapselte Mikropellet-Formulierung, hergestellt aus der Mikropellet-Formulierung nach Anspruch 2.

4.  Verfahren zur Herstellung des Danshen-Extrakts wie in Anspruch 1 definiert, das die folgenden Schritte umfasst:

    (1) Danshen wird mit Alkohol extrahiert und gefiltert, um eine Alkoholextraktionsflüssigkeit und einen Rückstand A zur späteren Verwendung zu ergeben; wobei der Alkohol vorzugsweise Ethanol mit einer Konzentration von 50 bis 100 % (v/v) in einer Menge des 2- bis 7-Fachen des Gewichts der Rohmedizin ist und die Extraktionszeit 0,5 bis 4 Stunden beträgt;

(2) der Rückstand A wird mit Wasser extrahiert, gefiltert, um eine Wasserextraktionsflüssigkeit und einen zu entsorgenden Rückstand B zu ergeben; wobei die Menge an zugesetztem Wasser vorzugsweise das 3- bis 7-Fache des Gewichts des Rückstands A beträgt und die Extraktionszeit 0,5 bis 4 Stunden beträgt;

(3) die Alkohol- bzw. Wasserextraktionsflüssigkeiten werden gekühlt und stehen gelassen, und beide Überstände werden entnommen, um den Alkohol- bzw. Wasserüberstand zu ergeben; wobei das Kühlen und Stehenlassen vorzugsweise die folgenden Schritte umfasst: die Extraktionsflüssigkeit wird während 20 bis 60 min gerührt und während 6 bis 24 Stunden stehen gelassen, wobei die Temperatur auf 15 °C oder darunter gesenkt wird, bevor der Überstand entnommen wird;

(4) der Wasserüberstand wird konzentriert, um eine konzentrierte Wasserextraktionsflüssigkeit zu haben; wobei der Wasserüberstand vorzugsweise auf eine relative Dichte von 1,10 bis 1,35 konzentriert wird, um die konzentrierte Wasserextraktionsflüssigkeit zu ergeben;

(5) die konzentrierte Wasserextraktionsflüssigkeit wird allmählich mit dem Alkoholüberstand versetzt, kombiniert und konzentriert, um eine gemischte konzentrierte Lösung zu erzielen;

(6) die gemischte konzentrierte Lösung wird allmählich mit gereinigtem Wasser versetzt und gut gemischt, um den Danshen-Extrakt zu ergeben; wobei die gemischte konzentrierte Lösung vorzugsweise mehrmals mit 10 L bis 100 L gereinigtem Wasser, jedes Mal 5 L bis 50 L, versetzt wird, gemischt wird und unter vermindertem Druck konzentriert wird, um den Extrakt mit einer relativen Dichte von 1,25 bis 1,35 bei $82,5 \pm 2,5$ °C zu ergeben.

5. Verfahren nach Anspruch 4, **dadurch gekennzeichnet, dass** das Verfahren die folgenden Schritte umfasst: Gemahlener Danshen wird genommen, mit $400 \pm 12$ L Ethanol ($90 \pm 0,5$ %) versetzt, während $90 \pm 5$ min abgekocht, durch ein Sieb mit einer Maschenweite von 200 gefiltert, gemischt und in einen Trennbehälter eingeführt; der resultierende Rückstand wird für die 2. Abkochung von $60 \pm 3$ min mit $500 \pm 15$ L Wasser versetzt, durch ein Sieb mit einer Maschenweite von 200 gefiltert, und der Rückstand wird verworfen; die Wasserextraktionsflüssigkeit wird gemischt und in unterschiedliche Behälter eingeführt; die gemischte Alkoholextraktionsflüssigkeit und die gemischte Wasserextraktionsflüssigkeit werden in unterschiedlichen Trennbehältern angeordnet, über die gefrorenes Wasser zur Kühlung geleitet wird, und stehen gelassen; nach 30 min Rühren wird die Extraktionsflüssigkeit innerhalb von 4 Stunden auf 15 °C oder darunter gekühlt und während 6 bis 24 Stunden stehen gelassen; der Wasserüberstand und der Alkoholüberstand werden in jeweilige Behälter überführt; zuerst wird der Wasserüberstand zu einer konzentrierten Wasserextraktionsflüssigkeit mit einer relativen Dichte von 1,25 bis 1,30 ($82,5 \pm 2,5$ °C) konzentriert, zu welcher der Alkoholüberstand allmählich zugesetzt wird, und wobei weiter gemischt und konzentriert wird; wobei während des Vorgangs des Konzentrierens die Dichte der konzentrierten Lösung nicht weniger als 1,15 beträgt; wenn die Dichte der gemischten konzentrierten Lösung $\geq$1,34 beträgt, werden in zwei Malen 10 L gereinigtes Wasser, jedes Mal 5 L ($45 \pm 5$ °C), zugesetzt, gemischt, auf eine relative Dichte von 1,33 bis 1,35 ($82,5 \pm 2.5$ °C) konzentriert und sofort durch ein Sieb mit einer Maschenweite von 40 gesiebt, um den Danshen-Extrakt zu ergeben.

6. Verfahren zur Herstellung eines Mikropellets, das den Danshen-Extrakt enthält, wie er in Anspruch 3 definiert wird, wobei es die folgenden Schritte umfasst:

(1) Danshen-Extrakt und blankes Mikropellet in einem Gewichtsverhältnis von 1:5 bis 5:1 werden zur späteren Verwendung genommen;

(2) Zuführen und Fluidisieren: das blanke Mikropellet mit Formulierungsdosierung wird in eine Fließbetttrocknungs-Beschichtungsmaschine absorbiert, um das blanke Mikropellet im Fließbett vollständig zu fluidisieren;

(3) Arzneimittelladung im frühen Stadium: bis die Materialtemperatur 40 bis 60 °C erreicht, wird eine Flüssigkeit mit einer Rate von 70 - 120 g/min versprüht und auf der Oberfläche des Mikropellets verteilt; bei der Flüssigkeit, so wie sie versprüht wird, handelt es sich um kleine Tröpfchen, die hergestellt werden, indem vorgemischter Danshen-Extrakt zerstäubt wird, der erhalten wird, indem Danshen-Extrakt mit Wasser verdünnt wird;

(4) Arzneimittelladungsstadium: wenn das Material 40 bis 60 °C mit einer Erhöhung des Durchmessers des Mikropellets aufweist, wird das Sprühvolumen allmählich erhöht; wobei der Durchmesser des Mikropellets und die Sprührate stufenweise erhöht werden, um ein Granulat mit einem Durchmesser von 0,5 bis 1,8 mm zu ergeben.

7. Verfahren nach Anspruch 6, **dadurch gekennzeichnet, dass** das Verfahren zusätzlich den Schritt (5) umfasst: nach dem Sprühen wird das Arzneimittelladungs-Mikropellet beschichtet, indem eine Beschichtungsflüssigkeit versprüht wird; wobei die Temperatur des Beschichtungsmaterials 40 bis 60 °C, die Sprührate 40 bis 300 g/min, die Beschichtungszeit 1 bis 4 Stunden und die Konzentration der Beschichtungsflüssigkeit 5 bis 25 % beträgt.

8. Verfahren nach Anspruch 7, **dadurch gekennzeichnet, dass** das Verfahren die folgenden Schritte umfasst:

(1) Danshen-Extrakt und blankes Mikropellet in einem Gewichtsverhältnis von 1:3 bis 3: 1, vorzugsweise 2:1 bis 1:1, werden zur späteren Verwendung genommen;

(2) Zuführen und Fluidisieren: mit einem Luftvolumen von 600 bis 1.500 m$^3$/h wird das blanke Mikropellet in eine Fließbetttrocknungs-Beschichtungsmaschine absorbiert, um das blanke Mikropellet im Fließbett vollständig zu fluidisieren; die Fluidisierung kann jedoch nicht zu stark sein, um einen Abrieb und ein Stauben des blanken Mikropellets zu verhindern; dadurch sollte das blanke Mikropellet mit den Tröpfchen beschichtet werden, die durch eine Sprühpistole versprüht werden;

(3) Arzneimittelladung im frühen Stadium: die Sprühpistole und der Sprühdruck werden eingestellt, um Flüssigkeiten in kleine Tröpfchen zu zerstäuben; der Extrakt wird durch eine Dosierpumpe zu der Sprühpistole geliefert; die Drücke des oberen und unteren Luftstroms werden auf 2,0 bis 3,0 bar bzw. 2,5 bis 3,5 bar eingestellt; die Temperatur des Materials beträgt 50 °C; bis die Temperatur 45 °C erreicht, wird mit dem Versprühen der Zufuhrlösung mit einer Rate von 120 g/min begonnen;

(4) Arzneimittelladungsstadium: wenn das Material mit einer Erhöhung des Durchmessers des Mikropellets 40 bis 60 °C aufweist, wird das Sprühvolumen allmählich erhöht, wobei die maximale Rate jedoch nicht mehr als 400 g/min beträgt; das Luftvolumen wird in Abhängigkeit von dem Fluidisierungszustand des Mikropellets eingestellt;

(5) Beschichtung: nach der Arzneimittelladung wird die Beschichtungsflüssigkeit direkt versprüht, um die Beschichtung durchzuführen; anfänglich wird die Materialtemperatur auf 50 °C und die Sprührate auf 80 g/min festgelegt; nach 20 min Beschichtung kann die Sprührate auf 80 bis 150 g/min eingestellt werden, die Materialtemperatur kann auf 40 bis 55 °C reguliert werden; die Infusionsrate, das Luftvolumen und die Materialtemperatur werden in Abhängigkeit von der Klebkraft des Mikropellets eingestellt;

vorzugsweise wird die Extraktmedizinlösung durch eine Verdünnung des Danshen-Extrakts mit Wasser hergestellt; wobei das Gewichtsverhältnis von Danshen-Extrakt zu Wasser 100 : 60 bis 100, vorzugsweise 100 : 70 bis 90, stärker bevorzugt 100 : 75 bis 85 beträgt.

9. Verfahren nach einem der Ansprüche 6 bis 8, **dadurch gekennzeichnet, dass** die Mikropellet-Formulierung die Mikropellet-Formulierung nach Anspruch 2 ist.

10. Danshen-Extrakte nach Anspruch 1 oder Mikropellet-Formulierung nach Anspruch 2 oder eingekapselte Mikropellet-Formulierung nach Anspruch 3 zur Verwendung bei der Verbesserung des Mikrokreislaufs oder bei der Senkung der Blutfette.

## Revendications

1. Extrait de sauge rouge (Salvia miltiorrhiza), **caractérisé en ce que** ledit extrait comprend les composants suivants en partie en poids : danshensu/acide rosmarinique:acide lithospermique:acide salvianolique B:cryptotanshinone:tanshinone IIA:stachyose = (0,5-16) : (0,5-15) : (0,5-15) : (5-140) : (0,5-25) : (1-50) : (150-600) ; de préférence lesdits composants en partie en poids : (1-8) : (1-8) : (1-8) : (10-70) : (1-10) : (2-20) : (250-500) ; de manière davantage préférée lesdits composants en partie en poids : (2-5) : (2-5) : (2-5) : (25-60) : (2-6) : (4-10) : (300-450) ; de manière encore davantage préférée lesdits composants en partie en poids : (2-4) : (2-4) : (2-4) : (25-30) : (2-5) : (4-10) : (330-400) ; de manière préférée entre toutes lesdits composants en partie en poids : 3:3:3:28:4:7:370.

2. Formulation de microgranulés comprenant ledit extrait de sauge rouge selon la revendication 1 en tant que principe actif ; de préférence la densité apparente dudit microgranulé est de 0,6 ~ 1,3 g/ml, la surface spécifique est de 0,005 ~ 0,05 m$^2$/g et la taille de particules est de 0,5 à 1,8 mm ; de manière davantage préférée la densité apparente dudit microgranulé est de 0,8 ~ 1,1 g/ml, la surface spécifique est de 0,01 ~ 0,03 m$^2$/g et la taille de particules est de 0,7 à 1,2 mm.

3. Formulation de microgranulés encapsulée préparée à partir de la formulation de microgranulés selon la revendication 2.

4. Procédé de préparation d'un extrait de sauge rouge qui comprend les étapes suivantes :

(1) la sauge rouge est extraite avec de l'alcool et filtrée pour donner un liquide d'extraction alcoolique et un résidu A pour une utilisation ultérieure ; de préférence ledit alcool est l'éthanol en une concentration de 50 ~

100 % (v/v) en une quantité de 2 ~ 7 fois le poids de médicament brut et une durée d'extraction de 0,5 à 4 heures ;

(2) le résidu A est extrait avec de l'eau, filtré pour donner un liquide d'extraction aqueux et le résidu B à jeter ; de préférence la quantité d'eau ajoutée est de 3 ~ 7 fois le poids du résidu A et la durée d'extraction est de 0,5 à 4 heures ;

(3) respectivement, les liquides d'extraction alcoolique et aqueux sont refroidis avant de pouvoir reposer puis les deux surnageants sont prélevés pour donner les surnageants alcoolique et aqueux respectivement ; de préférence ledit refroidissement et ledit repos comportent les étapes suivantes : le liquide d'extraction est agité pendant 20 ~ 60 minutes avant de pouvoir reposer pendant 6 ~ 24 heures avec la température réduite à 15 °C ou moins, avant que le surnageant ne soit retiré ;

(4) le surnageant aqueux est concentré pour avoir un liquide d'extraction aqueux concentré ; de préférence le surnageant aqueux est concentré à une densité relative de 1,10 ~ 1,35 pour donner un liquide d'extraction aqueux concentré ;

(5) au liquide d'extraction aqueux concentré est ajouté graduellement le surnageant alcoolique, avant la combinaison et la concentration pour donner une solution concentrée mixte ;

(6) à la solution concentrée mixte est ajoutée de l'eau purifiée, suivi d'un mélange rigoureux pour obtenir l'extrait de sauge rouge ; de préférence à la solution concentrée mixte sont ajoutés 10 l ~ 100 l d'eau purifiée plusieurs fois, 5 l à 50 l chaque fois, suivi du mélange et de la concentration sous pression réduite pour obtenir l'extrait avec une densité relative de 1,25 ~ 1,35 à 82,5 ± 2,5 °C.

5. Procédé selon la revendication 4, **caractérisé en ce que** ledit procédé comprend les étapes suivantes : de la sauge rouge broyée est prise, à laquelle sont ajoutés 400 ± 12 l d'éthanol (90 ± 0,5 %), décoctée pendant 90 ± 5 minutes, filtrée au travers d'un tamis de 200 meshes, mélangée et placée dans une cuve de séparation ; au résidu obtenu sont ajoutés 500 ± 15 l d'eau pour une seconde décoction de 60 ± 3 minutes, le résidu est filtré au travers d'un tamis de 200 meshes et le résidu est jeté ; le liquide d'extraction aqueux est mélangé et placé dans différentes cuves ; le liquide d'extraction alcoolique mixte et le liquide d'extraction aqueux mixte sont placés dans des cuves de séparation différentes, qui sont traversées par de l'eau glacée pour le refroidissement avant de laisser reposer ; après une agitation de 30 minutes, le liquide d'extraction est refroidi à 15 °C ou moins pendant 4 heures avant de pouvoir reposer pendant 6 ~ 24 heures ; le surnageant aqueux et le surnageant alcoolique sont transférés à des cuves respectives ; premièrement le surnageant aqueux est concentré en un liquide d'extraction aqueux concentré ayant une densité relative de 1,25 ~ 1,30 (82,5 ± 2,5°C), dans lequel le surnageant alcoolique est progressivement ajouté puis mélangé et concentré ; durant le processus de concentration, la densité de la solution concentrée n'est pas inférieure à 1,15 ; lorsque la densité de la solution mélangée est ≥ 1,34, 10 l d'eau purifiée sont ajoutés en deux fois, 5 l chaque fois (45 ± 5 °C), mélangés, concentrés à une densité relative de 1,33 ~ 1,35 (82,5 ± 2,5 °C), et immédiatement filtrés à travers un tamis de 40 meshes pour donner l'extrait de sauge rouge.

6. Procédé de préparation d'un microgranulé contenant un extrait de sauge rouge selon la revendication 3 qui comprend les étapes suivantes :

(1) l'extrait de sauge rouge et un microgranulé vierge en un rapport en poids de 1:5 ~ 5:1 sont pris pour une utilisation ultérieure ;

(2) alimentation et fluidisation : le microgranulé vierge de la formule posologique est absorbé dans une machine d'enrobage et de séchage à l'état fluidisé pour obtenir le microgranulé vierge totalement fluidisé en lit fluidisé ;

(3) chargement de médicament à un stade précoce : jusqu'à ce que la température du matériau atteigne 40 à 60 °C, un liquide est pulvérisé et répandu sur la surface du microgranulé à un taux de 70 à 120 g/minute ; ledit liquide tel que pulvérisé est des petites gouttelettes préparées par atomisation de l'extrait de sauge rouge prémélangé obtenu par dilution de l'extrait de sauge rouge avec de l'eau ;

(4) stade de chargement de médicament : lorsque le matériau est à 40 ~ 60 °C, avec une augmentation du diamètre du microgranulé, le volume de pulvérisation est progressivement accru ; le diamètre du microgranulé et le taux de pulvérisation sont accrus par gradient pour obtenir un granulé avec un diamètre de 0,5 à 1,8 mm.

7. Procédé selon la revendication 6, **caractérisé en ce que** ledit procédé comprend en outre l'étape (5) : après la pulvérisation, le microgranulé de chargement de médicament est enrobé par un liquide d'enrobage par pulvérisation ; la température du matériau d'enrobage est de 40 à 60 °C, le taux de pulvérisation est de 40 à 300 g/minute, la durée d'enrobage est de 1 à 4 heures, et la concentration du liquide d'enrobage est de 5 à 25 %.

8. Procédé selon la revendication 7, **caractérisé en ce que** ledit procédé comprend les étapes suivantes :

(1) l'extrait de sauge rouge et le microgranulé vierge en un rapport en poids de 1:3 ~ 3:1, de préférence de 2:1

~ 1:1, sont pris pour une utilisation ultérieure ;

(2) alimentation et fluidisation : à un volume d'air de 600 à 1 500 m$^3$/h, le microgranulé vierge est absorbé dans la machine d'enrobage et de séchage à l'état fluidisé pour obtenir le microgranulé vierge totalement fluidisé en lit fluidisé ; cependant, la fluidisation ne doit pas être trop sévère, de manière à prévenir l'abrasion et le poudrage du microgranulé vierge ; en raison de ceci, le microgranulé vierge doit être couvert avec les gouttelettes atomisées par le pulvérisateur ;

(3) chargement de médicament à un stade précoce : le pulvérisateur et la pression de pulvérisation sont ajustés pour faire s'atomiser les liquides en petites gouttelettes ; l'extrait est délivré au pulvérisateur par une pompe doseuse ; les pressions des buses d'air supérieure et inférieure sont respectivement définies à 2,0 ~ 3,0 bars et 2,5 ~ 3,5 bars ; la température du matériau est à 50 °C ; jusqu'à ce que la température atteigne 45 °C, la solution d'alimentation commence à se pulvériser à un taux de 120 g/min ;

(4) étape de chargement de médicament : lorsque le matériau est à 45 ~ 55 °C, avec une augmentation du diamètre du microgranulé, le volume de pulvérisation est progressivement accru, mais le taux maximal ne dépasse pas 400 g/minute ; le volume d'air est ajusté selon l'état fluidisé du microgranulé ;

(5) enrobage : après le chargement de médicament, le liquide d'enrobage est directement pulvérisé pour réaliser l'enrobage ; initialement, la température du matériau est définie à 50 °C et le taux de pulvérisation à 80 g/minute ; après 20 minutes d'enrobage, le taux de pulvérisation peut être ajusté à 80 à 150 g/minute, la température du matériau régulée à 40 à 55 °C ; le taux d'infusion, le volume d'air et la température du matériau sont ajustés en fonction de l'adhésion du microgranulé ;

de préférence, la solution d'extrait de médicament est préparée par dilution de l'extrait de sauge rouge avec de l'eau ; le rapport en poids de l'extrait de sauge rouge sur l'eau est de 100:160-100, de préférence de 100:70-90, de manière davantage préférée de 100:75-85.

**9.** Procédé selon l'une quelconque des revendications 6 à 8, **caractérisé en ce que** ladite formulation de microgranulés est la formulation de microgranulés selon la revendication 2.

**10.** Extraits de sauge rouge selon la revendication 1 ou la formulation de microgranulés selon la revendication 2 ou la formulation de microgranulés encapsulée selon la revendication 3 pour leur utilisation dans l'amélioration de la microcirculation ou la réduction des lipides sanguins.

(1)

(2)

(3)

(a)                                        (b)

Fig. 1

(a)                                        (b)

Fig. 2

concentration-mortality curve of Danshen extract E01

Fig. 3

Fig. 4

model group

0.08 μg/ml lovastatin group

189 μg/ml Danshen

630 μg/ml Danshen

1890 μg/ml Danshen

Fig. 5

strength of tail vascular fat staining

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1247855 A
- CN 1270809 A
- CN 01142288
- CN 1827130 A
- CN 102846705 A
- CN 102772487 A
- CN 103127220 A
- CN 101773546 A

**Non-patent literature cited in the description**

- **GUANHUA DU.** *Basic Medical Science and Clinics,* 2000, vol. 20 (5), 10-14
- *Chemical Pharmaceutical Bulletin,* 1989, vol. 37 (2), 340-344
- *PlantaMedica,* 1997, vol. 63, 22-26
- **CAI.** Study on Extraction And Refinement of Salvia Miltiorrhiza. *Chinese Archives of Traditional Chinese Medicine,* 2012, vol. 11 (30), 2565-2570